# EUROPEAN PATENT SPECIFICATION

(11) **EP 2 478 362 B1**
(45) Date of publication and mention of the grant of the patent: **11.05.2016**
(21) Application number: 10817999.5
(22) Date of filing: 20.09.2010
(51) Int. Cl.: G01N 33/53, A61K 31/015

(54) **USE OF THE SPARC MICROENVIRONMENT SIGNATURE IN THE TREATMENT OF CANCER**
VERWENDUNG DER SPARC-MIKROUMGEBUNGS-SIGNATUR BEI DER KREBSBEHANDLUNG
UTILISATION D'UNE SIGNATURE DE MICROENVIRONNEMENT SPARC DANS LE TRAITEMENT DU CANCER

(30) Priority: 18.09.2009 US 276969 P
(43) Date of publication of application: 25.07.2012
(73) Proprietor: Abraxis BioScience, LLC, Los Angeles, CA 90025 (US)
(72) Inventor: TRIEU, Vuong, Calabasas, California 91302 (US); DESAI, Neil, Los Angeles, California 90025 (US)
(74) Representative: Grünecker Patent- und Rechtsanwälte PartG mbB
(86) International application number: PCT/US2010/049545
(87) International publication number: WO 2011/035274

(56) References cited:
- WO-A2-2008/060651
- US-A1- 2007 054 271
- US-A1- 2008 255 035
- ANDREW H BECK ET AL: "The fibromatosis signature defines a robust stromal response in breast carcinoma", LABORATORY INVESTIGATION, vol. 88, no. 6, 1 June 2008 (2008-06-01), pages 591-601, XP55073836, ISSN: 0023-6837, DOI: 10.1038/labinvest.2008.31
- WATKINS G ET AL: "Increased levels of SPARC (osteonectin) in human breast cancer tissues and its association with clinical outcomes", PROSTAGLANDINS LEUKOTRIENES AND ESSENTIAL FATTY ACIDS, CHURCHILL LIVINGSTONE, EDINBURGH, vol. 72, no. 4, 1 April 2005 (2005-04-01), pages 267-272, XP004780199, ISSN: 0952-3278, DOI: 10.1016/J.PLEFA.2004.12.003
- MASSI D ET AL: "Osteonectin expression correlates with clinical outcome in thin cutaneous malignant melanomas", HUMAN PATHOLOGY, SAUNDERS, PHILADELPHIA, PA, US, vol. 30, no. 3, 1 March 1999 (1999-03-01), pages 339-344, XP008097964, ISSN: 0046-8177, DOI: 10.1016/S0046-8177(99)90014-X
- JEFFREY R INFANTE ET AL: "Peritumoral Fibroblast SPARC Expression and Patient Outcome With Resectable Pancreatic Adenocarcinoma", JOURNAL OF CLINICAL ONCOLOGY, AMERICAN SOCIETY OF CLINICAL ONCOLOGY, US, vol. 25, no. 3, 20 January 2007 (2007-01-20), pages 319-325, XP008148514, ISSN: 0732-183X, DOI: 10.1200/JCO.2006.07.8824

## Description

### RELATED CASES

This patent application claims the benefit of U.S. Provisional Patent Application No. 61/27,969, which was filed on September 18, 2009.

### BACKGROUND OF THE INVENTION

Secreted protein acidic and rich in cysteine (also known as osteonectin, BM40, or SPARC) (hereinafter "SPARC"), is a matrix-associated protein that elicits changes in cell shape, inhibits cell-cycle progression, and influences the synthesis of extracellular matrix (Bradshaw et al., Proc. Nat. Acad. Sci. USA 100: 6045-6050 (2003)). The murine SPARC gene was cloned in 1986 (Mason et al., EMBO J. 5: 1465-1472 (1986)) and a full-length human SPARC cDNA was cloned and sequenced in 1987 (Swaroop et al., Genomics 2: 37-47 (1988)). SPARC expression is developmentally regulated, and is predominantly expressed in tissues undergoing remodeling during normal development or in response to injury. For example, high levels of SPARC protein are expressed in developing bones and teeth (*see, e.g*., Lane et al., FASEB J., 8, 163 173 (1994); Yan & Sage, J. Histochem. Cytochem. 47:1495-1505 (1999)).

SPARC is upregulated in several aggressive cancers, but is absent in the corresponding normal tissues (Porter et al., J. Histochem. Cytochem., 43, 791 (1995)). SPARC expression is induced among a variety of tumors (e.g., bladder, liver, ovary, kidney, gut, and breast). In bladder cancer, for example, SPARC expression has been associated with advanced carcinoma. Invasive bladder tumors of stage T2 or greater have been shown to express higher levels of SPARC relative to bladder tumors of stage T1 (or less superficial tumors), and a poorer prognosis (*see, e.g.,* Yamanaka et al., J. Urology, 166, 2495 2499 (2001)). In meningiomas, SPARC expression has been associated only with invasive tumors (*see, e.g.,* Rempel et al., Clincal Cancer Res., 5, 237 241 (1999)). SPARC expression also has been detected in 74.5% of *in situ* invasive breast carcinoma lesions (*see, e.g.,* Bellahcene, et al., Am. J. Pathol., 146, 95 100 (1995)), and 54.2% of infiltrating ductal carcinoma of the breast (*see, e.g.,* Kim et al., J. Korean Med. Sci., 13, 652 657 (1998)). SPARC expression also has been associated with frequent microcalcification in breast cancer (*see, e.g.,* Bellahcene *et al., supra*), suggesting that SPARC expression may be responsible for the affinity of breast metastases for the bone.

Surprisingly, SPARC has also been shown to have anti-tumor activity in some systems. SPARC is a potent cell cycle inhibitor that arrests cells in mid-G1 (Yan & Sage, J. Histochem. Cytochem. 47:1495-1505 (1999)) and the inducible expression of SPARC has been shown to inhibit breast cancer cell proliferation in an in vitro model system (Dhanesuan et al., Breast Cancer Res. Treat. 75:73-85 (2002)). Similarly, exogenous SPARC can reduce the proliferation of both HOSE (human ovarian surface epithelial) and ovarian cancer cells in a concentration-dependent manner. In addition, SPARC induces apoptosis in ovarian cancer cells. Further, SPARC receptors on cells such as ovarian epithelial cells have been reported. It has been proposed that the binding of SPARC to its receptor is likely to trigger tissue-specific signaling pathways that mediate SPARC's tumor suppressing functions (Yiu et al., Am. J. Pathol. 159:609-622 (2001)). Purified SPARC has also been reported to inhibit angio-gnesis and impair neuroblastoma tumor growth in an in vivo xenograft model system (Chlenski et al., Cancer Res. 62:7357-7363 (2002)).

These seemlingly conflicting results may be due to SPARC's many forms, which result from differential splicing and post translational modifications of immature SPARC. As a result, e.g., fibroblast SPARC is a different molecule than platelet SPARC. In addition, SPARC is differentially glycosylated. (*See* Kaufman et al., Glycobiology 14(7): 609-619 (2004)). SPARC is readily degraded by a variety of proteases and appears to undergo turnover in extracellular environments. The turnover of SPARC by extracellular proteases results in the exposure of novel SPARC epitopes (Lane & Sage, FASEB J. 8 (2):163-173 (1994)). These factors result in a wide range of immunohistologic staining patterns. Each antibody can produce markedly different staining patterns.
The PCT publication WO 2008/060651 A2 ("the '651 Publication") provides methods for predicting or determining the response of a mammalian tumor to a chemotherapeutic agent and for treating a mammalian tumor comprising detecting and quantifying the SPARC protein or RNA in a sample isolated from the mammal. In some embodiments the '651 Publication uses immunohistochemistry to determine the amount of SPARC present.

Cancer is now primarily treated with one or a combination of three types of therapies: surgery, radiation, and chemotherapy. Surgery generally is only effective for treating the earlier stages of cancer. For more than 50% of individuals with cancer, by the time they are diagnosed they are no longer candidates for effective surgical treatment. Radiation therapy is only effective for individuals who present with clinically localized disease at early and middle stages of cancer, and is not effective for the late stages of cancer with metastasis.

Chemotherapy involves the disruption of cell replication or cell metabolism. Chemotherapy can be effective, but there are severe side effects, e.g., vomiting, low white blood cells (WBC), hair loss, weight loss and other toxic effects. Because of the extremely toxic side effects, many individuals with cancer cannot successfully finish a complete chemotherapy regime. Chemotherapy-induced side effects significantly impact the quality of life of the individual and may dramatically influence the individual's compliance with treatment. Additionally, adverse side effects associated with chemotherapeutic agents are generally the major dose-limiting toxicity (DLT) in the administration of these drugs. For example, mucositis is a major dose limiting toxicity for several anticancer agents, including the antimetabolite cytotoxic agents 5-FU, methotrexate, and antitumor antibiotics, such as doxorubicin. When severe, many of these chemotherapy-induced side effects may lead to hospitalization, or require treatment with analgesics for pain. Additionally, poor tolerance to chemotherapy can lead to death in some individuals with cancer.. The extreme side effects of anticancer drugs are caused by poor target specificity. The drugs circulate through most normal organs as well as the intended target, tumors. The poor target specificity that causes side effects also decreases the efficacy of chemotherapy because only a fraction of the drugs are correctly targeted. The efficacy of chemotherapy is further decreased by poor retention of the anti-cancer drugs within the target tumors.

Due to the severity and breadth of cancer, there is a great need for effective treatments of such diseases or disorders that overcome the shortcomings of surgery, chemotherapy, and radiation treatment. In particular, in view of the serious side effects associated with chemotherapy, there is a need to identify which tumors will or will not respond to chemotherapeutic regimens.

The invention described herein provides novel methods of treating cancer based on the exploitation of the heterogeneous immunohistology observed with different SPARC antibodies.

### BRIEF SUMMARY OF THE INVENTION

The invention provides the following embodiments as defined under items 1-11:
1. A chemotherapeutic agent for use in a chemotherapeutic regimen for treating a breast cancer or pancreatic cancer in a mammal, wherein the chemotherapeutic agent is to be administered to the mammal if the cancer SPARC microenvironment signature (SMS) satisfies the criteria of a predefined SMS, wherein the SMS is determined upon
   a. preparing a plurality of histologic sections of the cancer for immunohistology;
   b. immunostaining one or more of the histologic sections of the cancer with a first anti-SPARC antibody, wherein the first anti-SPARC antibody preferentially stains SPARC in cancer cells;
   c. immunostaining one or more of the histologic sections of the cancer with a second anti-SPARC antibody, wherein the second anti-SPARC antibody preferentially stains SPARC in fibroblasts;
   d. determining the staining of cancer cells, fibroblasts, inflammatory cells, acellular stroma/matrix, blood vessels, nerve tissue, and normal anatomy within the cancer, or any combinations thereof, with the first anti-SPARC antibody and the staining of cancer cells, fibroblasts, inflammatory cells, acellular stroma/matrix, blood vessels, nerve tissue, and normal anatomy within the cancer, or any combinations thereof, with the second antibody, thereby determining a SPARC microenvironment signature (SMS),
   and wherein the chemotherapeutic agent is nab-paclitaxel.
2. An in vitro method for predicting the response of a breast cancer or a pancreatic cancer in a mammal to a chemotherapeutic regimen comprising:
   a. preparing a plurality of histologic sections of the cancer to obtain a SPARC microenvironment signature (SMS);
   b. immunostaining one or more of the histologic sections of the cancer with a first anti-SPARC antibody, wherein the first anti-SPARC antibody preferentially stains SPARC in cancer cells;
   c. immunostaining one or more of the histologic sections of the cancer with a second anti-SPARC antibody, wherein the second anti-SPARC antibody preferentially stains SPARC in fibroblasts;
   d. determining the staining of cancer cells, fibroblasts, inflammatory cells, acellular stroma/matrix, blood vessels, nerve tissue, and normal anatomy within the cancer, or any combinations thereof, with the first anti-SPARC antibody and the staining of cancer cells, fibroblasts, inflammatory cells, acellular stroma/matrix, blood vessels, nerve tissue, and normal anatomy within the cancer, or any combinations thereof, with the second antibody;
   e. predicting a positive response to the chemotherapeutic regimen if a predefined SMS is demonstrated by the immunostaining,
   and wherein the chemotherapeutic regimen comprised nab-paclitaxel.
3. An in vitro method of predicting if a mammal with a breast cancer has a low risk of the progression of that cancer comprising:
   a. preparing a plurality of histologic sections of the cancer to obtain a SPARC microenvironment signature (SMS);
   b. immunostaining one or more of the histologic sections of the cancer with a first anti-SPARC antibody, wherein the first anti-SPARC antibody preferentially stains SPARC in cancer cells;
   c. immunostaining one or more of the histologic sections of the cancer with a second anti-SPARC antibody, wherein the second anti-SPARC antibody preferentially stains SPARC in fibroblasts;
   d. determining the staining of cancer cells, fibroblasts, inflammatory cells, acellular stroma/matrix, blood vessels, nerve tissue, and normal anatomy within the cancer, or any combinations thereof, with the first anti-SPARC antibody and the staining of cancer cells, fibroblasts, inflammatory cells, acellular stroma/matrix, blood vessels, nerve tissue, and normal anatomy within the cancer, or any combinations thereof, with the second antibody; and
   e. determining that there is a low risk of progression if the cancer SMS satisfies the criteria of a predefined SMS.
4. The chemotherapeutic agent for use according to item1 or the method according to item2, wherein the cancer is a pancreatic cancer.
5. The chemotherapeutic agent for use according to item 1 or the method according to item 2, wherein the cancer is a breast cancer.
6. The chemotherapeutic agent for use according to item 1, 4 or 5, or the method according to item 2, 3, 4 or 5, wherein the mammal is a human.
7. A therapeutic agent for use in a therapy for treating a breast cancer or pancreatic cancer from a first mammal, wherein the therapeutic agent is to be administered to the first mammal if the cancer from the first mammal's SMS maps to an outcome group that responds to the therapy, wherein the cancer from the first mammal is classified as a member of the outcome group upon
   a. determining two or more predictive SMS's for the therapy comprising:
      i. preparing a plurality of histologic sections of cancer from other mammals with known outcomes for the therapy;
      ii. immunostaining one or more of the histologic sections of each of the cancer from the other mammals with known outcomes for the therapy, with a first anti-SPARC antibody, wherein the first anti-SPARC antibody preferentially stains SPARC in cancer cells;
      iii. immunostaining one or more of the histologic sections of each of the cancer from the other mammals with known outcomes for the therapy, with a second anti-SPARC antibody, wherein the second anti-SPARC antibody preferentially stains SPARC in fibroblasts;
      iv. determining the immunostaining pattern of each of the cancers from other mammals with known outcomes for the therapy, for cancer cells, fibroblasts, inflammatory cells, acellular stroma/matrix, blood vessels, nerve tissue, and normal anatomy within the cancers from other mammals with known outcomes for the therapy, or any combinations thereof, with the first anti-SPARC antibody and the immunostaining of cancer cells, fibroblasts, inflammatory cells, acellular stroma/matrix, blood vessels, nerve tissue, and normal anatomy within the cancer from other mammals with known outcomes for the therapy, or any combinations thereof, with the second antibody, thereby determining the SMS of each cancer from the other mammals with known outcomes for the therapy;
      v. clustering the cancer SMS's of each cancer from the other mammals with known outcomes for the therapy into two or more outcome groups, wherein the SMS centroid of each outcome group defines a predictive SMS;
   b. determining the SMS of the cancer from the first mammal by a process comprising:
      i. preparing a plurality of histologic sections of the cancer from the first mammal;
      ii. immunostaining one or more of the histologic sections of the cancer from the first mammal with a first anti-SPARC antibody, wherein the first anti-SPARC antibody preferentially stains SPARC in cancer cells;
      iii. immunostaining one or more of the histologic sections of the cancer from the first mammal with a second anti-SPARC antibody, wherein the second anti-SPARC antibody preferentially stains SPARC in fibroblasts,
      iv. determining the immunostaining pattern of the cancer in the first mammal for cancer cells, fibroblasts, inflammatory cells, acellular stroma/matrix, blood vessels, nerve tissue, and normal anatomy within the cancer from the first mammal, or any combinations thereof, with the first anti-SPARC antibody and the immunostaining of the cancer from the first mammal of cancer cells, fibroblast, inflammatory cells, acellular stroma/matrix, blood vessels, nerve tissue, and normal anatomy within the cancer from the first mammal, or any combinations thereof, with the second antibody, thereby determining the first mammal's cancer SMS;
   c. determining the Euclidian distance of the cancer from the first mammal's SMS to the predictive SMS's determined in (a) and classifying the cancer from the first mammal as a member of the outcome group with the closest predictive SMS,
   and wherein the cancer from the first mammal is a breast cancer or a pancreatic cancer and the therapeutic agent is nab-paclitaxel.
8. The therapeutic agent according to item 7, wherein the cancers from the first mammal is of the same type as at the cancers from other mammals with known outcomes for the therapy.
9. The therapeutic agent according to item 7, wherein the cancer from the first mammal is a pancreatic cancer.
10. The therapeutic agent according to item 7, wherein the clustering of the cancer SMS's of each cancer from the other mammals with known outcomes for the therapy is performed by one or more of K-means, Self Organizing Maps and Hierarchical clustering.
11. The therapeutic agent according to item 7, wherein the cancer from the first mammal is a breast cancer.

The present disclosure provides the use of anti-SPARC antibodies for treating a tumor in a first mammal with a therapy comprising: determining two or more predictive SPARC Microenvironmental Signatures ("SMSs") for the therapy comprising: preparing a plurality of histologic sections of the tumors from other mammals with known outcomes for the therapy; immunostaining one or more histologic sections of each of the tumors from the other mammals, with known outcomes for the therapy, with a first anti-SPARC antibody, wherein the first anti-SPARC antibody preferentially stains SPARC in tumor cells; immunostaining one or more histologic sections of each of the tumors from the other mammals, with known outcomes for the therapy, with a second anti-SPARC antibody, wherein the second anti-SPARC antibody preferentially stains SPARC in fibroblasts; determining the immunostaining pattern of each of the tumors from other mammals, with known outcomes for the therapy, for the tumor cells, fibroblast, inflammatory cells, acellular stroma/matrix, blood vessels, nerve tissue, normal anatomy within the tumor or any combinations thereof with the first anti-SPARC antibody and the immunostaining of the tumor cells, fibroblast, inflammatory cells, acellular stroma/matrix, blood vessels, nerve tissue, normal anatomy within tumor or any combinations thereof with the second antibody, thereby determining the SMS of each tumor from the other mammals, with known outcomes for the therapy; clustering the tumor SMSs of each tumor from the other mammals, with known outcomes into two or more outcome groups, wherein the wherein the SMS centroid of the cluster from each outcome group defines a predictive SMS. Next, the SMS of the tumor from the first mammal is determined by a process comprising: preparing a plurality of histologic sections of the tumor from the first mammal; immunostaining one or more histologic sections of the tumor from the first mammal with a first anti-SPARC antibody, wherein the first anti-SPARC antibody preferentially stains SPARC in tumor cells; immunostaining one or more histologic sections of the tumor from the first mammal with a second anti-SPARC antibody, wherein the second anti-SPARC antibody preferentially stains SPARC in fibroblasts; determining the immunostaining pattern of the tumor in the first mammal for the tumor cells, fibroblast, inflammatory cells, acellular stroma/matrix, blood vessels, nerve tissue, normal anatomy within tumor or any combinations thereof with the first anti-SPARC antibody and the immunostaining of the tumor from the first mammal of the tumor cells, fibroblast, inflammatory cells, acellular stroma/matrix, blood vessels, nerve tissue, normal anatomy within tumor or any combinations thereof with the second antibody, thereby determining the first mammal's tumor SMS; and determine the Euclidian distance of the first mammal's tumor SMS to the predictive SMSs determined in (a) and classify the first mammal's tumor as a member of the outcome group with the closest predictive SMS. A therapeutically effective amount of the therapy to the first mammal if the first mammal's tumor SMS maps to an outcome group that responds to the therapy.
Embodiments in accordance with the invention include, wherein the same tumor type in the first mammal is the same as that in all of the "other mammals" studied. Any suitable therapy can be used included chemotherapies, radiation therapies, surgical thearapies, alternative therapies, and combinations thereof. Suitable therapies include those which, e.g., include nab-paclitaxel. Suitable tumors include breast cancer, pancreatic cancer, and melanoma. Clustering may done by any suitable method, including, e.g., K-means culstering, Self Organizing Maps and Hierarchical clustering.

The disclosure provides the use of anti SPARC antibodies for treating a tumor in a mammal with a chemotherapeutic regimen or other suitable therapy comprising:
a. Preparing a plurality of histologic sections of the tumor to obtain SPARC microenvironment signatures (SMS),
b. immunostaining a histologic section of the tumor with a first anti-SPARC antibody, wherein the first anti-SPARC antibody preferentially stains SPARC in tumor cells,
c. immunostaining a histologic section of the tumor with a second anti-SPARC antibody, wherein the second anti-SPARC antibody preferentially stains SPARC in fibroblasts,
d. determining the staining of the tumor cells, fibroblast, inflammatory cells, acellular stroma/matrix, blood vessels, nerve tissue, normal anatomy within tumor or any combinations thereof with the first anti-SPARC antibody and the staining of the tumor cells, fibroblast, inflammatory cells, acellular stroma/matrix, blood vessels, nerve tissue, normal anatomy within tumor or any combinations thereof with the second antibody,
e. administering a therapeutically effective amount of the therapy if the SMS determined meets the criteria of a predefined SMS that indicates the use of the chemotherapeutic regimen or other suitable therapy.

Predefined SMSs provided by the invention that indicate the use of therapies include those comprising immunostaining with at least 49% of the stroma staining positive with first antibody and at least a fibroblast score of 66, fibroblast intensity of 41, tumor intensity of 26, inflammatory cells intensity of 51, inflammatory cells score of 55, blood vessel % of 33, tumor score of 54, blood vessel intensity of 64, fibroblast % of 54, blood vessel intensity of 64, inflammatory cells % of 43, and stroma score of 62 staining with the second antibody, wherein the therapy is a regimen comprising nab-paclitaxel and the tumor is pancreatic cancer. *i.e.,* employing an SMS comprising these 16 criteria from SPARC's expression in the tumor to indicate that a therapy should be used. Such Predefined SMSs also in accordance with the invention and in this group of Predefined SMSs include those comprised of only 2-15 these criteria of SPARC's expression in the tumor.

Those of ordinary skill will ready recognize that additional predefined SMSs can be identified by cluster analysis of other series of tumors.

The disclosure also provides kits for predicting the response of a tumor in a mammal to a chemotherapeutic regimen or other suitable therapy comprising:
a. an immunostain with a first anti-SPARC antibody, wherein the first anti-SPARC antibody preferentially stains SPARC in tumor cells, and
b. an immunostain with a second anti-SPARC antibody, wherein the second anti-SPARC antibody preferentially stains SPARC in fibroblasts.

The disclosure also provides in vitro methods of predicting response to chemotherapies or other suitable therapies and whether a mammal with a tumor has a low risk of the progression or death from a tumor comprising: Preparing a plurality of histologic sections of the tumor to obtain SPARC microenvironment signature (SMS), immunostaining a histologic section of the tumor with a first anti-SPARC antibody, wherein the first anti-SPARC antibody preferentially stains SPARC in tumor cells, immunostaining a histologic section of the tumor with a second anti-SPARC antibody, wherein the second anti-SPARC antibody preferentially stains SPARC in fibroblasts, determining the staining of the tumor cells, fibroblasts, inflammatory cells, acellular stroma/matrix, blood vessels, nerve tissue, normal anatomy within the tumor or any combinations thereof with the first anti-SPARC antibody and the staining of the tumor cells, fibroblasts, inflammatory cells, acellular stroma/matrix, blood vessels, nerve tissue, normal anatomy within the tumor or any combinations thereof with the second antibody, and predicting positive a response to chemotherapy or that there is a low risk of progression or death if there is a predefined SMS.

The disclosure also provides in vitro methods for predicting the response of a tumor in a mammal to a chemotherapeutic regimen comprising- response defined as but not limited to pathological response, overall survival, or progression free survival: immunostaining a histologic section of the tumor with an anti-SPARC antibody, wherein the anti-SPARC antibody recognizes the epitope recognized by the MAB941 monoclonal antibody, and predicting a poor response to the chemotherapeutic regimen if there is staining of the tumor cells in the histologic section with the anti-SPARC antibody. In particular, the invention provides methods for predicting the response of a pancreatic carcinoma to chemotherapeutic regimen, wherein the chemotherapeutic regimen comprises administering an albumin bound nanoparticle paclitaxel and gemcitabine.

The disclosure also provides in vitro methods for predicting the response of a tumor in a mammal to a chemotherapeutic regimen comprising: immunostaining a histologic section of the tumor with an anti-SPARC antibody that recognizes the imunodominant epitopes recognized by the AF941 polyconal antibody, and predicting a positive response to the chemotherapeutic regimen if there is staining of the tumor cells in the histologic section with the anti-SPARC antibody. In particular, the disclosure provides in vitro methods for predicting the response of the tumor to chemotherapeutic regimen, wherein the chemotherapeutic regimen comprises administering an albumin bound nanoparticle paclitaxel and gemcitabine.

Any one of the uses or in vitro methods provided by the invention include methods wherein the mammal is a human patient.

### BRIEF DESCRIPTION OF THE SEVERAL VIEWS OF THE DRAWING(S)

FIG. 1 depicts different patterns of SPARC immunostaining generated by two different anti-SPARC antibodies, monoclonal (A), polyclonal (B).
FIG. 2 depicts survival curves for breast cancer patients treated with a *nab-*paclitaxel based regimen and either expressing the D staining pattern or not.
FIG. 3 depicts a heat diagram from a K-means clustering of progression free survival (PFS) data in breast cancer patients.
FIG. 4A-C depict survival curves reflecting the effect of TN (A), ER (B), and PR (C) status on progression free survival (PFS) in breast cancer.
FIG. 5 depicts survival curves reflecting the effect of the SMS (SPARC microenvironment signature) and TN status on PFS in breast cancer.
FIG. 6 depicts survival curves reflecting the effect of the SMS and ER status on PFS in breast cancer.
FIG 7 depicts a survival curve reflecting the effect of the SMS and PR status on PFS in breast cancer.
FIG. 8 depicts a heat diagram from a clustering of response data in breast cancer patients using five survival categories.
FIG. 9 depicts a heat diagram from a clustering of response data in breast cancer patients using two survival categories.
FIG. 10 depicts a heat diagram from a K-means clustering of PFS data in pancreatic cancer dividing the patients into good prognosis and bad prognosis SMS groups.
FIG. 11 depicts survival curves for PFS in (A) and overall survival (OS) (B) in pancreatic cancer based on SMS.
FIG. 12 depicts survival curves for PFS in (A) and overall survival (OS) (B) pancreatic cancer based on CA19 status.
FIG 13 depicts a survival curve reflecting the effect of the SMS and CA 19 status on PFS in pancreatic cancer.
FIG 14 depicts a survival curve reflecting the effect of the SMS and CA 19 status on OS in pancreatic cancer.
FIG. 15 depicts a heat diagram from a K-means clustering of PFS data in melanoma patients dividing patients into good and bad PFS groups.
FIG. 16 depicts survival curves for PFS in (A) and overall survival (OS) (B) melanoma based on SMS.

### DETAILED DESCRIPTION OF THE INVENTION

As used herein, the term "tumor" refers to any neoplastic growth, proliferation or cell mass whether benign or malignant (cancerous), whether a primary site lesion or metastases. As used herein, the term "cancer" refers to a proliferative disorder caused or characterized by the proliferation of cells which have lost susceptibility to normal growth control. Cancers of the same tissue type usually originate in the same tissue, and may be divided into different subtypes based on their biological characteristics. Four general categories of cancers are carcinoma (epithelial tissue derived), sarcoma (connective tissue or mesodermal derived), leukemia (blood-forming tissue derived) and lymphoma (lymph tissue derived). Over 200 different types of cancers are known, and every organ and tissue of the body may be affected. Specific examples of cancers that do not limit the definition of cancer may include melanoma, leukemia, astrocytoma, glioblastoma, retinoblastoma, lymphoma, glioma, Hodgkins' lymphoma and chronic lymphocyte leukemia. Examples of organs and tissues that may be affected by various cancers include pancreas, breast, thyroid, ovary, uterus, testis, prostate, thyroid, pituitary gland, adrenal gland, kidney, stomach, esophagus or rectum, head and neck, bone, nervous system, skin, blood, nasopharyngeal tissue, lung, urinary tract, cervix, vagina, exocrine glands and endocrine glands. Alternatively, a cancer may be multicentric or of unknown primary site (CUPS).

As used herein, a 'cancerous cell' refers to a cell that has undergone a transformation event and whose growth is no longer regulated to the same extent as before said transformation event.

As used herein, a "medicament" is a composition capable of producing an effect that may be administered to a patient or test subject. The effect may be chemical, biological or physical, and the patient or test subject may be human, or a non-human animal, such as a rodent or transgenic mouse. The composition may include small organic or inorganic molecules with distinct molecular composition made synthetically, found in nature, or of partial synthetic origin. Included in this group are nucleotides, nucleic acids, amino acids, peptides, polypeptides, proteins, or complexes comprising at least one of these entities, The medicament may be comprised of the effective composition alone or in combination with a pharmaceutically acceptable excipient.

As used herein, a "pharmaceutically acceptable excipient" includes any and all solvents, dispersion media, coatings, antibacterial, antimicrobial or antifungal agents, isotonic and absorption delaying agents, and the like that are physiologically compatible. The excipient may be suitable for intravenous, intraperitoneal, intramuscular, intrathecal or oral administration. The excipient may include sterile aqueous solutions or dispersions for extemporaneous preparation of sterile injectable solutions or dispersion. Use of such media for preparation of medicaments is known in the art.

As used herein, a "effective amount" or a "pharmacologically effective amount" or a "therapeutically effective amount" of a medicament, drug or therapy refers to an amount which upon administration it rearches concentrations in the therapeutic level of the medicament, drug or therapy delivered over the term that it is used. This may be dependent on mode of delivery, time period of the dosage, age, weight, general health, sex and diet of the subject receiving the medicament. The determination of what dose is a "pharmacologically effective amount" requires routine optimization which is within the capabilities of one of ordinary skill in the art. A cancer or cancerous cell may be described as "sensitive to" or "resistant to" a given therapeutic regimen or chemotherapeutic agent based on the ability of the regimen to kill cancer cells or decrease tumor size, reduce overall cancer growth (i.e. through reduction of angio elements), and/or inhibit metastasis. Cancer cells that are resistant to a therapeutic regimen may not respond to the regimen and may continue to proliferate. Cancer cells that are sensitive to a therapeutic regimen may respond to the regimen resulting in cell death, a reduction in tumor size, reduced overall growth (tumor burden) or inhibition of metastasis.

As used herein, a "therapeutic regimen" or "therapy" refers to the administration of at least one agent which is harmful to cancerous cells. Suitable therapeutic regimens for use in accordance with the invention include, but are not limited to, "chemotherapeutic regimens," "radiotherapeutic regimens," "alternative therapeutic regimen" and combinations thereof.

As used herein, "chemotherapy" refers to the administration of at least one chemotherapy agent which is harmful to destroy cancerous cells. There are a myriad of such chemotherapy agents available to a clinician. Chemotherapy agents may be administered to a subject in a single bolus dose, or may be administered in smaller doses over time. A single chemotherapeutic agent may be used (single-agent therapy) or more than one agent may be used in combination (combination therapy). Chemotherapy may be used alone to treat some types of cancer. Alternatively, chemotherapy may be used in combination with other types of treatment, for example, radiotherapy or alternative therapies (for example immunotherapy) as described herein. Additionally, a chemosensitizer may be administered as a combination therapy with a chemotherapy agent.

As used herein, a "chemotherapeutic agent" or "anticancer drug" refers to a medicament that may be used to treat cancer, and generally has the ability to kill cancerous cells directly. Examples of chemotherapeutic agents include alkylating agents, antimetabolites, natural products, hormones and antagonists, and miscellaneous agents. Examples of alternate names are indicated in brackets. Examples of alkylating agents include nitrogen mustards such as mechlorethamine, cyclophosphamide, ifosfamide, melphalan (L-sarcolysin) and chlorambucil; ethylenimines and methylmelamines such as hexamethylmelamine and thiotepa; alkyl sulfonates such as busulfan; nitrosoureas such as carmustine (BCNU), semustine (methyl-CCNU), lomustine (CCNU) and streptozocin (streptozotocin); DNA synthesis antagonists such as estramustine phosphate; and triazines such as dacarbazine (DTIC, dimethyl-triazenoimidazolecarboxamide) and temozolomide . Examples of antimetabolites include folic acid analogs such as methotrexate (amethopterin); pyrimidine analogs such as fluorouracin (5-fluorouracil, 5-FU, 5FU), floxuridine (fluorodeoxyuridine, FUdR), cytarabine (cytosine arabinoside) and gemcitabine; purine analogs such as mercaptopurine (6-mercaptopurine, 6-MP), thioguanine (6-thioguanine, TG) and pentostatin (2'-deoxycoformycin, deoxycoformycin), cladribine and fludarabine; and topoisomerase inhibitors such as amsacrine. Examples of natural products include vinca alkaloids such as vinblastine (VLB) and vincristine; taxanes such as paclitaxel and docetaxel (Taxotere); epipodophyllotoxins such as etoposide and teniposide; camptothecins such as topotecan and irinotecan; antibiotics such as dactinomycin (actinomycin D), daunorubicin (daunomycin, rubidomycin), doxorubicin, bleomycin, mitomycin (mitomycin C), idarubicin, epirubicin; enzymes such as L-asparaginase; and biological response modifiers such as interferon alpha and interlelukin 2. Examples of hormones and antagonists include luteinising releasing hormone agonists such as buserelin; adrenocorticosteroids such as prednisone and related preparations; progestins such as hydroxyprogesterone caproate, medroxyprogesterone acetate and megestrol acetate; estrogens such as diethylstilbestrol and ethinyl estradiol and related preparations; estrogen antagonists such as tamoxifen and anastrozole; androgens such as testosterone propionate and fluoxymesterone and related preparations; androgen antagonists such as flutamide and bicalutamide; and gonadotropin-releasing hormone analogs such as leuprolide. Examples of miscellaneous agents include thalidomide; platinum coordination complexes such as cisplatin (cis-DDP), oxaliplatin and carboplatin; anthracenediones such as mitoxantrone; substituted ureas such as hydroxyurea; methylhydrazine derivatives such as procarbazine (N-methylhydrazine, MIH); adrenocortical suppressants such as mitotane (o,p'-DDD) and aminoglutethimide; RXR agonists such as bexarotene; and tyrosine kinase inhibitors such as imatinib. Alternate names and trade-names of these and additional examples of chemotherapeutic agents, and their methods of use including dosing and administration regimens, will be known to a person versed in the art. In particular, suitable chemotherapeutic agents for use in accordance with the invention include, without limitation, nanoparticle albumin-bound paclitaxels.

Abraxane™, also known as ABI-007, is a preferred chemotherapeutic agent. Abraxane™ is an albumin-nanoparticle formulation of paclitaxel. The use of an albumin nanoparticle as a vehicle results in the formation of a colloid when reconstituted with saline. Based on clinical studies, it has been shown that the use of Abraxane™ is characterized by reduced hypersensitivity reactions as compared with Taxol.™ Accordingly, premedication is not required for patients receiving Abraxane™.

Another advantage of the albumin-nanoparticle formulation is that by excluding toxic emulsifiers it is possible to administer higher doses of paclitaxel at more frequent intervals than is possible with Taxol™. The potential exists that enhanced efficacy could be seen in solid tumors as a consequence of (i) higher tolerable doses (300 mg/m²), (ii) longer half-life, (iii) prolonged local tumor availability and/or (iv) sustained in vivo release Abraxane™.

A positive response is defined as including, but not limited, to pathological response (reduction in tumor size or burden), overall survival, or progression free survival as shown by an improvement of the metric by at least 5 %, preferably by at least 10%, more preferably by at least 15%, even more preferably by at least 20%, most preferably by at least 25% or more. Alternatively, the metric shows an improvement by a statistically significant amount in comparison with no or prior or alternative therapy.

A negative response includes, but is not limited to pathological progression, decreased overall or progression free survival.

As used herein, the term "radiotherapeutic regimen" or "radiotherapy" refers to the administration of radiation to kill cancerous cells. Radiation interacts with various molecules within the cell, but the primary target, which results in cell death is the deoxyribonucleic acid (DNA). However, radiotherapy often also results in damage to the cellular and nuclear membranes and other organelles. DNA damage usually involves single and double strand breaks in the sugar-phosphate backbone. Furthermore, there can be crosslinking of DNA and proteins, which can disrupt cell function. Depending on the radiation type, the mechanism of DNA damage may vary as does the relative biologic effectiveness. For example, heavy particles (i.e. protons, neutrons) damage DNA directly and have a greater relative biologic effectiveness. Electromagnetic radiation results in indirect ionization acting through short-lived, hydroxyl free radicals produced primarily by the ionization of cellular water. Clinical applications of radiation consist of external beam radiation (from an outside source) and brachytherapy (using a source of radiation implanted or inserted into the patient). External beam radiation consists of X-rays and/or gamma rays, while brachytherapy employs radioactive nuclei that decay and emit alpha particles, or beta particles along with a gamma ray.

Radiotherapy may further be used in combination chemotherapy, with the chemotherapeutic agent acting as a radiosensitizer. The specific choice of radiotherapy suited to an individual patient may be determined by a skilled person at the point of care, taking into consideration the tissue and stage of the cancer.

As used herein, the term "alternative therapeutic regimen" or "alternative therapy" may include for example, biologic response modifiers (including polypeptide-, carbohydrate-, and lipid-biologic response modifiers), toxins, lectins, antiangiogenic agents, receptor tyrosine kinase inhibitors (for example Iressa™ (gefitinib), Tarceva™ (erlotinib), Erbitux™ (cetuximab), imatinib mesilate (Gleevec™), proteosome inhibitors (for example bortezomib, Velcade™); VEGFR2 inhibitors such as PTK787 (ZK222584), aurora kinase inhibitors (for example ZM447439); mammalian target of rapamycin (mTOR) inhibitors, cyclooxygenase-2 (COX-2) inhibitors, rapamycin inhibitors (for example sirolimus, Rapamune™); farnesyltransferase inhibitors (for example tipifarnib, Zarnestra); matrix metalloproteinase inhibitors (for example BAY 12-9566; sulfated polysaccharide tecogalan); angiogenesis inhibitors (for example Avastin™ (bevacizumab); analogues of fumagillin such as TNP-4; carboxyaminotriazole; BB-94 and BB-2516; thalidomide; interleukin-12; linomide; peptide fragments; and antibodies to vascular growth factors and vascular growth factor receptors); platelet derived growth factor receptor inhibitors, protein kinase C inhibitors, mitogen-activated kinase inhibitors, mitogen-activated protein kinase kinase inhibitors, Rous sarcoma virus transforming oncogene (SRC) inhibitors, histonedeacetylase inhibitors, small hypoxia-inducible factor inhibitors, hedgehog inhibitors, and TGF-β signalling inhibitors. Furthermore, an immunotherapeutic agent would also be considered an alternative therapeutic regimen. Examples include chemokines, chemotaxins, cytokines, interleukins, or tissue factor. Suitable immunotherapeutic agents also include serum or gamma globulin containing preformed antibodies; nonspecific immunostimulating adjuvants; active specific immunotherapy; and adoptive immunotherapy. In addition, alternative therapies may include other biological-based chemical entities such as polynucleotides, including antisense molecules, polypeptides, antibodies, gene therapy vectors and the like. Such alternative therapeutics may be administered alone or in combination, or in combination with other therapeutic regimens described herein. Alternate names and trade-names of these agents used in alternative therapeutic regimens and additional examples of agents used in alternative therapeutic regimens, and their methods of use including dosing and administration regimens, will be known to a physician versed in the art. Furthermore, methods of use of chemotherapeutic agents and other agents used in alternative therapeutic regimens in combination therapies, including dosing and administration regimens, will also be known to a person versed in the art.

In particular, suitable alternative therapeutic regimens include, without limitation, antibodies to molecules on the surface of cancer cells such as antibodies to Her2 (e.g., Trastuzumab), EGF or EGF Receptors, VEGF (e.g., Bevacizumab) or VEGF Receptors, CD20, and the like. The therapeutic agent may further comprise any antibody or antibody fragment which mediates one or more of complement activation, cell mediated cytotoxicity, inducing apoptosis, inducing cell death, and opsinization. For example, such an antibody fragment may be a complete or partial Fc domain.

As used herein, the term "histologic section" refers to a thin section of a tissue sample suitable for mounting on a microscope slide and staining with any suitable protocol. As used herein, "immunostaining a histologic section" refers to the staining of the cells and intracellular matrix of the histologic section resulting from the binding of antibodies to components of the cells are intracellular matrix. As used herein, to "predominantly" or "preferentially" stain a structure, e.g., a cancer cell over a fibroblast, the immunostaining of the preferentially stained structure in the histologic section should be of an intensity graded by a pathologist by any suitable system, including, e.g., 3/3 when observed microscopically by those of ordinary skill, well all other structures stain with only an intensity of 1/3 or show 0/3 (no staining).

As used herein, the term "epitope" refers to the three-dimensional structure bound by an antibody, and in particular the amino acid sequence targeted by the antibody. As used herein, the term "epitope recognized by the MAB941 monoclonal antibody" refers to the amino acid sequence in SPARC bound by the MAB941 monoclonal anybody. (SPARC monoclonal antibody (R&D Systems, Minneapolis, MN), catalog # MAB941)

As used herein, "imunodominant epitopes" refers to the three-dimensional structures bound with the greatest collective avidity by the antibodies in polyclonal antisera. In particular, the epitopes responsible for the pattern of staining in immunostaining protocol employing that polyclonal antisera. As used herein, the term "imunodominant SPARC epitopes recognized by the AF941 polyconal antibody refers" to the SPARC peptides and amino acid sequences found with the greatest avidity by the AF941 polyconal antisera. Accordingly, binding to and staining of these SPARC peptides and amino acid sequences results and the majority of immunostaining observed. (SPARC polyclonal antibody (R&D Systems, Minneapolis, MN), catalog # AF941)

By "antibodies" it is meant without limitation, monoclonal antibodies, polyclonal antibodies, dimers, multimers, multispecific antibodies (e.g., bispecific antibodies). Antibodies may be murine, human, humanized, chimeric, or derived from other species. An antibody is a protein generated by the immune system that is capable of recognizing and binding to a specific antigen. A target antigen generally has numerous binding sites, also called epitopes, recognized by CDRs on multiple antibodies. Each antibody that specifically binds to a different epitope has a different structure. Thus, one antigen may have more than one corresponding antibody.

An antibody includes a full-length immunoglobulin molecule or an immunologically active portion of a full-length immunoglobulin molecule, i.e., a molecule that contains an antigen binding site that immunospecifically binds an antigen of a target of interest or part thereof. Targets include, cancer cells or other cells that produce autoimmune antibodies associated with an autoimmune disease.

The immunoglobulins disclosed herein can be of any class (e.g., IgG, IgE, IgM, IgD, and IgA) or subclass (e.g., IgG1, IgG2, IgG3, IgG4, IgA1 and IgA2) of immunoglobulin molecule. The immunoglobulins can be derived from any species.

"Antibody fragments" comprise a portion of a full length antibody, which maintain the desired biological activity. "Antibody fragments" are generally the antigen binding or variable region thereof. Examples of antibody fragments include Fab, Fab', F(ab')2, and Fv fragments; diabodies; linear antibodies; fragments produced by a Fab expression library, anti-idiotypic (anti-Id) antibodies, CDR (complementary determining region), and epitope-binding fragments of any of the above which immunospecifically bind to cancer cell antigens, viral antigens or microbial antigens, single-chain antibody molecules; and multispecific antibodies formed from antibody fragments.

The monoclonal antibodies referenced herein specifically include "chimeric" antibodies in which a portion of the heavy and/or light chain is identical with or homologous to corresponding sequences in antibodies derived from a particular species or belonging to a particular antibody class or subclass, while the remainder of the chain(s) is identical with or homologous to corresponding sequences in antibodies derived from another species or belonging to another antibody class or subclass, as well as fragments of such antibodies, so long as they exhibit the desired biological activity (U.S. Pat. No. 4,816,567). Chimeric antibodies of interest herein include "primatized" antibodies comprising variable domain antigen-binding sequences derived from a non-human primate (e.g., Old World Monkey or Ape) and human constant region sequences.

"Antibody-dependent cell-mediated cytotoxicity" and "ADCC" refer to a cell-mediated reaction in which nonspecific cytotoxic cells that express Fc receptors (FcRs) (e.g., Natural Killer (NK) cells, neutrophils, and macrophages) recognize bound antibody on a target cell and subsequently cause lysis of the target cell. The primary cells for mediating ADCC, NK cells, express Fc.γ.RIII only, whereas monocytes express FcγRI, FcγRII and FcγRIII. To assess ADCC activity of a molecule of interest, an in vitro ADCC assay may be performed (U.S. Pat. No. 5,003,621; U.S. Pat. No. 5,821,337). Useful effector cells for such assays include peripheral blood mononuclear cells (PBMC) and Natural Killer (NK) cells.

An antibody which "induces cell death" is one which causes a viable cell to become nonviable. Cell death in vitro may be determined in the absence of complement and immune effector cells to distinguish cell death induced by antibody-dependent cell-mediated cytotoxicity (ADCC) or complement dependent cytotoxicity (CDC). Thus, the assay for cell death may be performed using heat inactivated serum (i.e., in the absence of complement) and in the absence of immune effector cells. To determine whether the antibody is able to induce cell death, loss of membrane integrity as evaluated by uptake of propidium iodide (PI), trypan blue or 7AAD can be assessed relative to untreated cells. Cell death-inducing antibodies are those which induce PI uptake in the PI uptake assay in BT474 cells.

An antibody which "induces apoptosis" is one which induces programmed cell death as determined by binding of annexin V, fragmentation of DNA, cell shrinkage, dilation of endoplasmic reticulum, cell fragmentation, and/or formation of membrane vesicles (called apoptotic bodies).

As used herein, a "chemosensitizer" or "sensitizer" is a medicament that may enhance the therapeutic effect of a chemotherapeutic agent, radiotherapy treatment or alternative therapeutic regimen, and therefore improve efficacy of such treatment or agent. The sensitivity or resistance of a tumor or cancerous cell to treatment may also be measured in an animal, such as a human or rodent, by, e.g., measuring the tumor size, tumor burden or incidence of metastases over a period of time. For example, about 2, about 3, about 4 or about 6 months for a human and about 2-4, about 3-5, or about 4-6 weeks for a mouse. A composition or a method of treatment may sensitize a tumor or cancerous cell's response to a therapeutic treatment if the increase in treatment sensitivity or the reduction in resistance is about 10% or more, for example, about 30%, about 40%, about 50%, about 60%, about 70%, about 80%, or more, to about 2- fold, about 3-fold, about 4-fold, about 5-fold, about 10-fold, about 15-fold, about 20-fold or more, compared to treatment sensitivity or resistance in the absence of such composition or method. The determination of sensitivity or resistance to a therapeutic treatment is routine in the art and within the skill of a person versed in the art.

The terms "'peptide," "polypeptide," and "protein" may be used interchangeably, and refer to a compound comprised of at least two amino acid residues covalently linked by peptide bonds or modified peptide bonds, for example peptide isosteres (modified peptide bonds) that may provide additional desired properties to the peptide, such as increased half-life. A peptide may comprise at least two amino acids. The amino acids comprising a peptide or protein described herein may also be modified either by natural processes, such as posttranslational processing, or by chemical modification techniques which are well known in the art. Modifications can occur anywhere in a peptide, including the peptide backbone, the amino acid side-chains and the amino or carboxyl termini. It is understood that the same type of modification may be present in the same or varying degrees at several sites in a given peptide.

A tissue array can be made and stained by any suitable method known to those of ordinary skill in the art. For example, tissue cores from formalin-fixed, paraffin-embedded tumor blocks (2 cores from the most representative areas per block) can be arrayed (Beecher Instruments, Silver Spring, Md) to create a tissue microarray of cores measuring 2.0 mm each and were placed on positively charged slides. Slides with specimens are then placed in a 60 °C oven for 1 hour, cooled, deparaffinized, and rehydrated through xylenes and graded ethanol solutions to water. All slides are quenched for 5 minutes in a 3% hydrogen peroxide solution in water to block for endogenous peroxidase. Antigen retrieval can be performed by any suitable technique, e.g., a heat method in which the specimens were placed in a citric acid solution, pH 6.1 (code S1699, Dako, Carpinteria, Calif) for 20 minutes at 94 °C using a vegetable steamer, then cooled for 15 minutes. Slides are then placed on a Dako Autostainer immunostaining system for use with immunohistochemistry utilizing suitable antibodies. This method is based on the consecutive application of (1) a primary antibody against the antigen to be localized, (2) biotinylated linking antibody, (3) enzyme-conjugated streptavidin, and (4) substrate chromogen (DAB). Slides were then counterstained in Richard-Allan hematoxylin (Kalamazoo, Mich), dehydrated through graded ethanol solutions, and topped with a coverslip.

A 2-color double immunostain can be performed using any suitable protocol known to those of ordinary skill in the art. For example, without limitation, paraffin-embedded tissue blocks can cut at 4 µm and placed on positively charged slides. Slides with specimens were then placed in a 60 °C oven for 1 hour, cooled, deparaffinized, and rehydrated through xylenes and graded ethanol solutions to water. All slides are then quenched for 5 minutes in a 3% hydrogen peroxide solution in water to block for endogenous peroxidase. Antigen retrieval can be performed using any suitable protocol known to those of ordinary skill in the art. For example, by a heat method in which the specimens were placed in a citric acid solution (pH 6.1) for 25 minutes (as compared with 20 minutes for the individual antibodies mentioned previously) at 94 °C and cooled for 15 minutes using a vegetable steamer. Slides can then, e.g. , be placed on a Dako Autostainer immunostaining system, for use with immunhistochemistry.

The first primary antibody is incubated for 30 minutes at room temperature. The detection system, EnVision+ dual link (Dako, code K4061), is incubated for 30 minutes. Lastly, DAB chromogen is added. Before the second primary antibody is applied, serum-free protein block is added (Dako, code X0909) to minimize background and crossover between primary antibodies. The second primary antibody is incubated for 1 hour at room temperature. The EnVision+ dual link (Dako, code K4061) was used again as the detection system and incubated for 30 minutes. NovaRED (Vector Laboratories, Burlingame, Calif) can be used with second primary so that the staining by the two antibodies can be easily differentiated. Slides are then counterstained in Richard-Allan hematoxylin, dehydrated through graded ethanol solutions, and topped with a coverslip.

Suitable anti-SPARC antibodies can be identified using tissue microarrays to assay for the correct distribution of tumor and fibroblast SPARC staining. Mono and polyclonal antibodies made by standard techniques known in the art can be used.

Tissue microarrays comprising duplicate 0.6-mm cores from the selected blocks can be constructed using a Beecher Instruments Micro Tissue Arrayer. Four-micrometer-thick sections can be cut from completed array blocks and transferred to silanized glass slides. Sections from these arrays then can be stained with hematoxylin and eosin to assess adequacy. Microwave antigen retrieval can consist of placing the slides in 10 mM citrate buffer (pH 6.0) in a pressure cooker (Nordic Ware) and microwaving on high power until the buffer had boiled under pressure for 4 minutes. At this point, microwaving was stopped and the slides were incubated in the pressure cooker for a further 20 minutes, after which they were removed and rinsed. Proteinase antigen retrieval consisted of a 4-minute incubation in protease-1 solution (Ventana) according to the supplier's recommended protocol.

Epitope mapping can also be done using standard techniques known in the art. For example, the protocols from "Epitope Mapping," Chapter 11, in Using Antibodies by Ed Harlow and David Lane. Cold Spring Harbor Laboratory Press, Cold Spring Harbor, NY, USA, 1999. By mapping the epitopes, epitope-specific antibodies can be readily generated by standard techniques.

The methodology for determining the SPARC Microenvironment Signature (SMS) by Immunostaining histologic sections of the tumor with a first anti-SPARC antibody, wherein the first anti-SPARC antibody preferentially stains SPARC in tumor cells and with a second anti-SPARC antibody, wherein the second anti-SPARC antibody preferentially stains SPARC in fibroblasts. Seven components of SPARC expression were determined with the two different antibodies: tumor cells, fibroblasts, inflammatory cells, acellular stroma/matrix (stroma), blood vessels, nerves and the other normal anatomy within the tumor. The percent of cells stained in each field, the intensity of staining (0-4) and an score (dependant variable) for each of the components of the tumor were determined (total variables per patient: 7 components x 2 antibodies x 3 scores = 42 variables scored.)

The scoring combined the percent positive cells and staining intensity. The score was negative if no cells or none of the component stained positive. The score was "weakly positive" if <10% of the cells were positive whatever the intensity of staining, the intensity was 2+ or less and <20% of the cells were positive, or the intensity was 1+and <30% of the cells were positive. The score was "moderately positive" if the intensity was 4+ and 10-40% of the cells were positive, the intensity was 3+ and 10-50% of the cells were positive, the intensity was 2+ and 20-70% of the cells were positive, or the intensity was 4+ or less and 10-40% of the cells were positive or the intensity was 1+ or less and >30% of the cells were positive. The score was "strongly positive" if the intensity was 4+ and >40% of the cells were positive, or the intensity was 3+ and >50% of the cells were positive, the intensity was 2+ and >70% of the cells were positive.

This data was mined using the clustering programs in the Elementspring™ software suite and Nexus™ array analysis programs. In addition, ANOVA or t-test (unpaired) statistics were determined for parameters that clustering suggested had discriminating power for various outcome parameters.

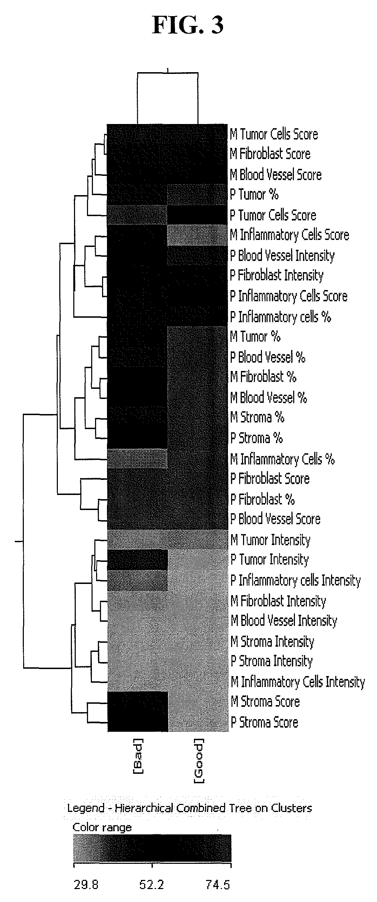

Hierarchical clustering is an extensively used data mining technique which provides a good 'first pass' analysis of data. It involves using one of several techniques iteratively, starting with one datapoint (i.e., measured parameter value) or "element," and combining elements with their nearest neighbor, gradually building clusters and associations of clusters. The final result is a hierarchical tree (e.g., FIG. 3). Distance between clusters is defined by the distance between their average expression patterns. A visual representation of the clusters is created in the form of a hierarchical tree, or dendrogram, familiar and easily understood by all biologists. The tree structure makes it easy to visually see how similar the expression patterns are between elements or sets of elements.

Non-hierarchical clustering techniques group N number of elements into K clusters. Two examples are K-Means clustering and Self Organizing Maps. K-means clustering begins with a predefined (K) number of clusters, or "centroids" and involves a three step process. First, elements are randomly assigned to a centroid. Second the mean inter and intra-cluster distances are then calculated. Finally, elements are moved from one cluster to another. Steps two and three are repeated until intra-cluster distance is minimized and inter-cluster distance in maximized, typically resulting in K round shaped clusters. New elements are grouped in the cluster with the nearest centroid. A centroid is the average of all the points in the cluster. K-means clustering excels at clustering elements where the number of groups is known. For example, a dataset containing cancerous and non-cancerous tissues could be analyzed according to K-means clustering to identify 2 groups of genes: those that change with cancer and those that do not.

Self Organizing Maps (SOM) are generated via neural network techniques to iteratively map nodes into n-dimensional "element space." This technique incorporates prior knowledge because a partial structure is imposed (the number of clusters and dimensionality must be assigned) prior to analysis. Then, random vectors are created and added to each node. Next, the distance between the vectors and a randomly selected gene are calculated. The vector closest to the gene is updated, making it more like the element's vector. The process is repeated thousands of times until no more changes can be made. This process converts large dimensional element space into something more manageable and understandable.

By the SPARC Microenvironmental Signature (SMS) it meant the pattern of staining with two anti-SPARC antibodies as indicated by the histologic location, intensity, and frequency of immunostaining with each antibody. By "clustering" it is meant the use of any suitable clustering method to group SMSs based on their clinical outcomes and identify the SMS components that contribute to distinguishing one group from another. Suitable methods include, e.g., K-means, Self Organizing Maps and Hierarchical clustering (all of which can be performed by commercially available software known to those of ordinary skill.) A "centroid" is the range of parameters that defines a cluster group. In this application in refers specifically to the SMS component values which distinguish different SMS groups, e.g., the criteria for being classified as a "responder." Assignment to an outcome group is the process of determining which centroid best represents the data available that defines your group.

While all clustering techniques excel under certain conditions they also have limitations, which will be know to those skilled in the art. For example, hierarchical clustering imposes a rigid relational structure on the data which may or may not reflect reality. K-means clustering and SOM generation require a predetermined number of clusters. This works well in certain situations, but for blind, exploratory data analysis, like determining gene relationships, the proper number of clusters cannot be determined ahead of time. K-means clustering has an additional limitation in that it produces fairly round clusters, resulting in inaccurate identification of close or geometrically shaped clusters. Lastly, although clustering shows an association between groups of elements, no conclusions can be drawn about relationships between elements within a cluster, such as a direction of action.

Reducing the number of elements is an important step which is desirably performed before the above described classification methods can be applied. This should be done so as to preserve as much discriminant information as possible to improve the learning accuracy. Properly defined elements should have the same expression pattern for all samples of the same class, and have different expression patterns from samples belonging to different classes. The "nearest shrunken centroid" method for class prediction uses "shrunken" centroids as prototypes for each class and identifies subsets of elements that best characterize each class. the method "shrinks" each of the class centroids toward the overall centroid for all classes by comparison to a threshold value. This shrinkage makes the classification more accurate by eliminating the effect of noisy elements and as a result automatically selects elements. The element profile of a new sample is compared to each of these class centroids. The class whose centroid that it is closest to, in squared distance, is the predicted class for that new sample.

There are two factors to consider in selecting proper elements for classifications: the distance within a class and the distance between classes. When element levels for all samples in the same class are fairly consistent with a small variance, but are largely different among samples of different classes, the element is considered a good candidate for classification. The gene has discriminating information for different classes. In the nearest shrunken centroid method, variance within a class was further taken into consideration to measure the goodness of a gene within class. The difference between a class centroid and the overall centroid for an element is divided by the variance within each class to give a greater weight to elements with lower variance. A threshold value is applied to the resulting normalized class centroid differences. If it is small for all classes, it is set to zero, meaning the element is eliminated. This reduces the number of elements that are used in the final predictive model.

Association rules can be used to identify the relationships between elements, relationships between a gene and several other groups of elements, and ultimately may indicate a particular treatment action. The first step is to discretize the data and convert it to a Boolean or tertiary notation. Then a cut-off value is established relative to which data is categorized as up regulated or down regulated. Up regulated genes, with values higher than the cut-off value, are assigned a value of '1.' Down regulated genes, with values below the cut-off value, are assigned a value of '0'. Alternatively, two cut-off values could be assigned, and genes could be categorized as up regulated (and assigned the value of 1), down regulated (and assigned the value of -1) or unchanged (and assigned the value of 0).

[0038]Any suitable dose of angiogenesis inhibitor may be used, e.g., Avastin administered at a dose of from about 5 mg/kg to about 15 mg/kg with a dosing cycle of at least 1 week.

Hydrophobic chemotherapeutic agents have an HLB (HLB is hydrophilic/lipophilic balance number) of 1.0 or less, preferably 2.0 or less, most preferably 5.0 or less, and include, e.g. the agents epothilone, docetaxel, paclitaxel. Microtubule inhibitor such as taxanes include epothilone, docetaxel, paclitaxel, and combinations thereof. "Combinations thereof" refers to both the administration of dosage forms including more than one drug, for example, docetaxel and paclitaxel, as well as the sequential but, temporally distinct, administration of epothilone, docetaxel and paclitaxel (e.g., the use of docetaxel in one cycle and paclitaxel in the next). Particularly preferred chemotherapeutic agents comprise particles of protein-bound drug, including but not limited to, wherein the protein making up the protein-bound drug particles comprises albumin including wherein more than 50% of the chemotherapeutic agent is in nanoparticle form. Most preferably the chemotherapeutic agent comprises particles of albumin-bound paclitaxel, such as, e.g., Abraxane™. Suitable nanoparticle formulations are not limited to those that comprise at least about 50% of the active agent in nanoparticle form. Other suitable nanoparticle formulations comprise at least about 60%, preferably at least about 70%, more preferably at least about 80%, or even more preferably at least about 90% of the active agent in nanoparticle form. Moreover, such nanoparticle formulations can most preferably comprise at least about 95% to at least about 98% of the active agent in nanoparticle form.

Suitable therapies for Her2 positive breast cancer also include regimens comprising six cycles of:neoadjuvant nab-Paclitaxel at 125 mg/m² on days 1, 8, 15 of each 28 day cycle,
carboplatin AUC6 on day 1 of each 28 day cycle; Trastuzumab with a 4 mg/kg load followed by 2 mg/kg/wk, and Bevacizumab at 5 mg/kg/wk; followed by surgical removal of the primary tumor; and post-operative therapeutically effective amounts of Trastuzumab and Bevacizumab for 52 weeks. Suitable therapies for Her2 negative breast cancer include, e.g., preoperative therapy comprising 6 cycles of 14 days with nab-Paclitaxel (175 mg/m²), gemcitabine (2000 mg/m²), and epirubicin (50 mg/m²); followed by surgical removal; and postoperative therapy comprising (4 cycles of 14 days) and nab-Paclitaxel (220 mg/m²) + gemcitabine (2000 mg/m²).

The following examples further illustrate the invention but, of course, should not be construed as in any way limiting its scope.

### EXAMPLE 1

The purpose of this study was to evaluate which SPARC isoforms and functions in the tumor microenvironement are responsible for patient outcomes and, in particular, to determine if there were correlations between patterns of SPARC immunostaining and patient outcomes with a nanoparticulate albumin-bound (*nab*) paclitaxel (*i.e.,* Abraxane^{®}).

*nab*-Paclitaxel can utilize endogenous pathways of albumin transport to enter tumor cells, including endothelial cell gp60-albumin receptor transport and binding to SPARC secreted by tumors. Initial preclinical studies and a small retrospective clinical study in head and neck cancer suggested that increased endogenous SPARC in tumor tissue may predict a favorable response to *nab*-paclitaxel treatment (Desai et al. 2009, Trans Onc. 2, 59-64).

Four prospective studies examined if SPARC tumor immunostaining patterns, *i.e.,* the "SPARC microenvironment signatures" (SMS), could discriminate patients with low and high risks of recurrence when treated with *nab*-paclitaxel regimens

The outcome of patients from the four clinical trials were evaluated (Table 1).

**Table 1. Clinical Trials That Provided Specimens and Outcome Data**

| **Study** | **Indication** | **Phase** | **No. Pts** | **No. Pts with SPARC IHC** | **Regimen** |
|---|---|---|---|---|---|
| N057E | Unresectable Stage IV Melanoma | II | 76 | 40 | *nab*-paclitaxel (100-150 mg/m²) wkly 3/4 carboplatin (AUC 2) wkly 3/4 |
| CA040 | Metastatic Pancreatic Cancer | I/II | 63 | 37 | *nab*-paclitaxel (100-150 mg/m²) wkly 3/4 gemcitabine (1000 mg/m2) wkly 3/4 |
| BRE73 | Neoadjuvant Breast Cancer (HER2-) | II | 123 | 83 | Preoperative : (6 cycles of 14 days) *nab*-paclitaxel (175 mg/m²) + gemcitabine (2000 mg/m2) + epirubicin (50 mg/m²) Postoperative: (4 cycles of 14 days) nab-paclitaxel (220 mg/m²) + gemcitabine (2000 mg/m²) |
| BRE83 | Neoadjuvant Breast Cancer (HER2+) | II | 30 | 30 | Preoperative: (6 cycles of 28 days) *nab*-paclitaxel (100 mg/m²) wkly ¾ + carboplatin (AUC6) + trastuzumab (4 mg/kg load, then 2 mg/kg/wk) +bevacizumab (5 mg/kg/wk) |
| | | | | | Post-operative maintenance (1yr) : |
| | | | | | trastuzumab (6 mg/kg) q3wk |
| | | | | | bevacizumab (15 mg/kg) q3wk |

Overall, this method is based on the consecutive application of (1) a primary antibody against the antigen to be localized, (2) biotinylated linking antibody, (3) enzyme-conjugated streptavidin, and (4) substrate chromogen (DAB). Slides are then counterstained in Richard-Allan hematoxylin (Kalamazoo, MI), dehydrated through graded ethanol solutions, and topped with a coverslip. All slides were stained using automated staining equipment (Dako Cytomation Autostainer, Dako, Carpinteria, CA).

The immunostaining in this example was performed as described below. A series of antibodies were evaluated against SPARC. Detailed immunohistologic evaluation was performed by a pathologist certified by the American Board of Pathology. Staining scores were assigned on scale of 0-4+, 4+ being the most positive. As it was not known which components of the tumor are important for SPARC's activity, a breakdown of the various components was performed, including staining in the tumor, blood vessels, fibroblasts, stromal cells, inflammatory cells, and the normal anatomy.

Tissue cores from formalin-fixed, paraffin-embedded tumor blocks (2 cores from the most representative areas per block) are arrayed (Beecher Instruments, Silver Spring, Md) to create a tissue microarray of cores measuring 2.0 mm each and are placed on positively charged slides. Slides with specimens are placed in a 60 °C oven for 1 hour, cooled, deparaffinized, and rehydrated through xylenes and graded ethanol solutions to water. All slides are quenched for 5 minutes in a 3% hydrogen peroxide solution in water to block for endogenous peroxidase.

Antigen retrieval is performed if no staining is seen and with the staining of normal tissue in the same field serving as an internal positive control. Antigen retrieval is performed by a heat method in which the specimens are placed in a citric acid solution, pH 6.1 (code S1699, Dako, Carpinteria, CA) for 20 minutes at 94 °C using a vegetable steamer, then cooled for 15 minutes. Slides are then placed on an immunostaining system such as the Dako Cytomation Autostainer (Dako, Carpinteria, CA) for use with immunohistochemistry utilizing suitable antibodies.

Two antibodies with differential affinity for SPARC were identified for this study, a monoclonal antibody (indicated hereinafter by "M") (SPARC monoclonal antibody (R&D Systems, Minneapolis, MN), catalog # MAB941 Lot # ECH045011 diluted 1:100 in a tris based diluent) and a polyclonal antibody (indicated hereinafter by "P") (SPARC polyclonal antibody (R&D Systems, Minneapolis, MN), catalog # AF941 Lot # EWN04 diluted 1:50 in a tris based diluents). Histologic sections of tumors were prepared on slides and stained using a standard immunostaining protocol. Briefly, tissue cores from formalin-fixed, paraffin-embedded tumor blocks (2 cores from the most representative areas per block) were arrayed (Beecher Instruments, Silver Spring, Md) to create a tissue microarray of cores measuring 2.0 mm each and were placed on positively charged slides. Slides with specimens were then placed in a 60 °C oven for 1 hour, cooled, deparaffinized, and rehydrated through xylenes and graded ethanol solutions to water. All slides were then quenched for 5 minutes in a 3% hydrogen peroxide solution in water to block for endogenous peroxidase. Antigen retrieval is performed by a heat method in which the specimens are placed in a citric acid solution (pH 6.1) for 25 minutes (as compared with 20 minutes for the individual antibodies mentioned previously) at 94 °C and cooled for 15 minutes using a vegetable steamer. Slides are then placed on an immunostaining system (Dako, Carpinteria, CA), for use with immunhistochemistry.

All slides were quenched for 5 minutes in a 3% hydrogen peroxide solution in water to block for endogenous peroxidase. After a buffer rinse, slides were incubated with antibody M or a negative control reagent for 30 minutes. A mouse horseradish peroxidase polymer kit (Mouse MACH 3 HRP Polymer Kit, Biocare Medical, Concord, CA) was incubated for 20 minutes per reagent. After another buffer rinse, DAB chromogen (Dako, Carpinteria, CA) was applied for 10 minutes. Hematoxylin was used to counterstain the slides. The same protocol was used for immunostaining specimens with antibody P, although an avidin-biotin detection kit (Biocare Medical, Concord, CA), incubated for 15 minutes per reagent, was used in place of the HRP detection kit.

Detailed pathological evaluation of SPARC expression in a series of tumors was performed by a board certified pathologist. The level of SPARC expression, as determined by immunohistochemistry, was scored for different tumor components. Scores were assigned to the level of SPARC expression on scale of 0-3, with 3 being the most positive score, as is commonly done in the art and well known to those of ordinary skill in the art. The monoclonal and polyclonal antibodies used detected different patterns of SPARC expression as shown in Table 2.

**Table 2. M and P Immunostaining Profiles.**

| | **Tumor** | | | **Fibroblast** | | |
|---|---|---|---|---|---|---|
| | **Antibody P** | **Antibody M** | | **Antibody P** | **Antibody M** | |
| **Breast** | 30/106 | 35/106 | p = ns | 82/107 | 26/107 | p < 0.0001 |
| **Pancreas** | 20/36 | 7/36 | p = 0.0031 | 18/29 | 5/29 | p = 0.0011 |
| **Melanoma** | 30/41 | 20/41 | p = 0.0408 | 19/33 | 14/33 | p = ns |

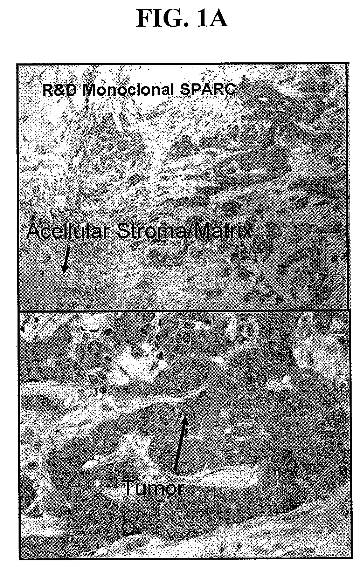

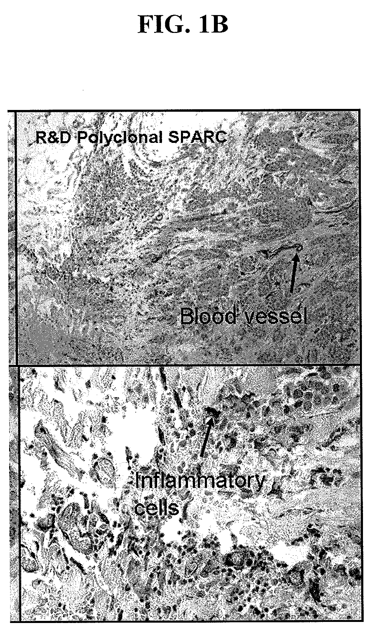

The polyclonal antibody demonstrated preferential staining of fibroblast associated SPARC, while the monoclonal anybody preferential stained tumor associated SPARC. (FIG. 1). From these staining preferences the following patterns were established analyzed for their predictive value in a series of tumors:
A, when 3+ was found in any of the components.
B, when 3+ was found in any of the components with the monoclonal anti-SPARC antibody.
C, when 3+ was found in any of the components with the monoclonal anti-SPARC antibody.
D, when 3+ was found in tumor cells with both anti-SPARC antibodies.
E, when 3+ was found in fibroblasts with both anti-SPARC antibodies.
Logistic regression and proportional hazard will be used to identify any correlations betweens SMS and response, progression free survival (PFS), and overall survival (OS) to SPARC staining pattern in various tumors.

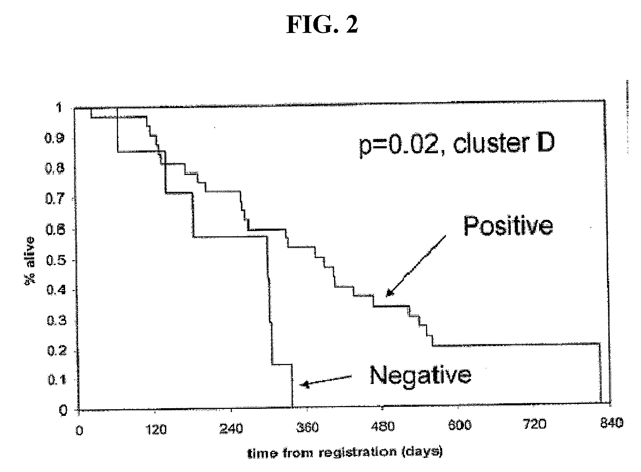

The first tumor set analyzed was a phase II trial of carboplatin and *nab*-paclitaxel (*a.k.a.,* ABI-007) in patients with unresectable stage IV melanoma (the N057E study). There was a statistically significant correlation between the D pattern and better overall survival (FIG. 2).

The second set of tumors was from patients with advanced pancreatic adenocarcinoma who had been treated with *nab*-paclitaxel doses (the CA040 study). The 32 patients studied had a full range of responses (Table 3. Response Rates).

**Table 3. Response Rates**

| **Response** | **CR** | **PR** | **SD** | **PD** |
|---|---|---|---|---|
| N of 32 pts | 2 (6%) | 14 (44%) | 14 (44%) | 2 (6%) |

| | | | | |
|---|---|---|---|---|
| (*CR, Complete Response; PR, Partial Response; SD, No Response and Stable Disease; PD, No Response and Progressive Disease) | | | | |

Staining of the tumor with the polyclonal antibody was predictive of responsiveness to therapy in this second set of tumors (advanced pancreatic cancer) (one tail t-test, p = 0.027). In addition, staining of the tumor cells with the monoclonal antibody predicted a worse overall survival and progression free survival. Further, B pattern staining was predictive of the worst progression free survival with this regimen in these patients with pancreatic adenocarcinoma.

This Example demonstrates that SPARC immunohistochemistry is a fruitful method for predicting response to *nab*-paclitaxel based chemotherapies.

### EXAMPLE 2

A more systematic analysis of the staining pattern data from SPARC immunostaining was undertaken to identify patterns which produced prognostic information, Staining pattern data from the same tumor sets studied in Example 1 were mined using various forms of cluster analysis to identify the most distinguishing components of SPARC expression (as indicated by the immunostaining pattern) for response, progression free survival (PFS), and overall survival (OS) to SPARC staining pattern in various tumors. As noted above, the patterns which emerged as prognostically significant are referred to as "SPARC microenvironment signatures" ("SMS")

SPARC expression was determined with the two different antibodies in seven tumor components: tumor cells, fibroblasts, inflammatory cells, acellular stroma/matrix (stroma), blood vessels, nerves and the other normal anatomy within the tumor. The percent of cells stained, the intensity of staining (0-4) and a "score" was then determined for each of the tumor components. The " score" combined the percent of stained cells and the staining intensity. The score was "negative" if no cells or none of the components stained were positive. The score was "weakly positive" if ≤20% of the cells were positive the intensity was 2+ or less, and also "weakly positive" if ≤30% of the cells were positive or the intensity was 1+ or less. The score was "moderately positive" if the intensity was 4+ and 10-40% of the cells were positive, the intensity was 3+ and 10-50% of the cells were positive, the intensity was 2+ and 20-70% of the cells were positive, or the intensity was 4+ or less and 20-40% of the cells were positive or the intensity was 1+ or less and >30% of the cells were positive. The score was "strongly positive" if the intensity was 4+ and >40% of the cells were positive, or the intensity was 3+ and >50% of the cells were positive, the intensity was 2+ and >70% of the cells were positive.

This data was mined using the clustering programs in the Elementspring^{®} software suite and Nexus^{®} array analysis programs. In addition, ANOVA or t-test (unpaired) statistics were determined for parameters that clustering indicated to have discriminating power.

SMS patterns were identified in the staining data from the BRE 73 breast cancer study. K-Means Cluster Analysis distinguished patients based on immunstaining who had superior PFS. PFS at 24 months was 56% for patients showing "bad" SPARC pattern as opposed to 91% PFS in the patients with a "good" SPARC good pattern.

Moreover, parameters were identified that separated these patients into prognostic groups (Table 4) ("Cut Off Value" is the value required to be classified in the good prognostic group) (*see also,* FIG. 3).

**Table 4. SMS Components for PFS in Breast Cancer**

| **SMS Component** | **Cut Off Value** | **p-Value** |
|---|---|---|
| P Inflammatory cells % | ≥ 50% | <0.0001 |
| M Tumor % | ≥ 70% | <0.0001 |
| P Blood Vessel % | ≥ 70% | <0.0001 |
| M Fibroblast % | ≥ 70% | <0.0001 |
| M Blood Vessel % | ≥ 70% | <0.0001 |
| M Stroma % | ≥ 70% | <0.0001 |
| P Stroma % | ≥ 70% | <0.0001 |
| M Inflammatory cells % | ≥ 70% | <0.0001 |

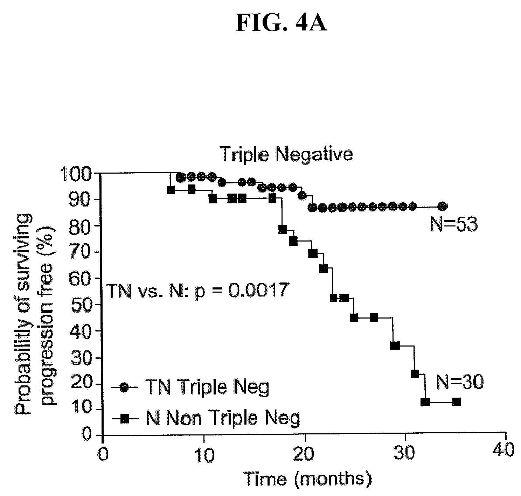

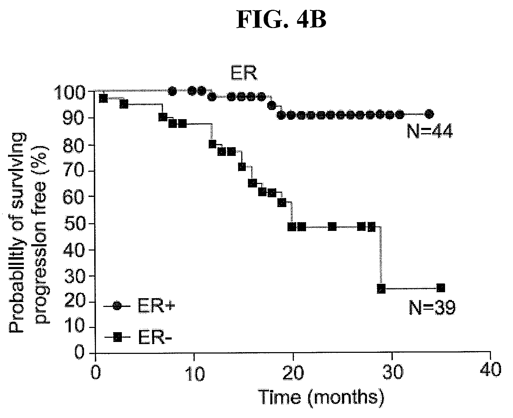

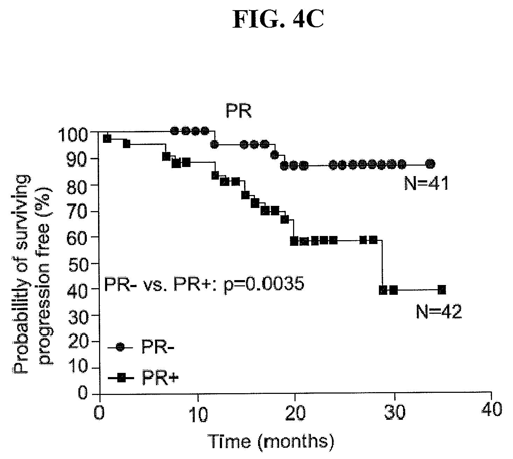

As expected estrogen receptor (ER), progesterone receptor (PR), and Triple Negative (TN) status predicted PFS (FIG. 4A-C). Surprisingly, the SMS functioned as an independent risk factor (*i.e.,* independent of the known risk factors, ER/PR/Triple Negative status (Table 5))

**Table 5. SMS and Known Risk Factors in Breast Cancer**

| | **ER-(N=39)** | **PR-(N=42)** | **Triple Negative (N=30)** |
|---|---|---|---|
| **SPARC Good SMS with a known risk factor** | 15/37 (41%) | 16/37 (43%) | 10/37 (27%) |
| **SPARC Bad SMS with a known risk factor** | 24/46 (52%) | 26/46 (57%) | 20/46 (43%) |
| ***statistics*** | *p*=*ns* | *p*=*ns* | *p*=*ns* |

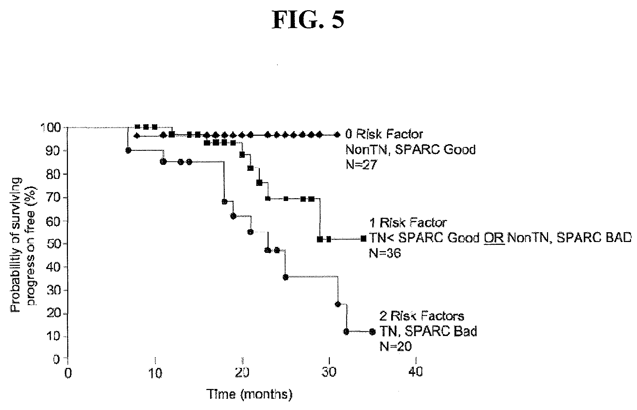

Further, when the SMS was added to known risk factors it improved stratification or discrimination between low and high risk groups based PFS for the *nab*-paclitaxel based regimen studied (FIG. 5-7). PFS at 24 months was significantly different between groups with 0, 1, and 2 risk factors. But, the addition of SPARC SMS to Triple Negative status further discriminated patients with low risk (0 risk factors) and high risk (≥2 risk factors) (log rank p values for different number of risk factors: 0 factors versus 2 factors, p = 0.0009; 1 factor vs 2 factors, p = 0.039.) (FIG. 5). Also, a group with 1 risk factor was found to be distinct and with intermediate risk.

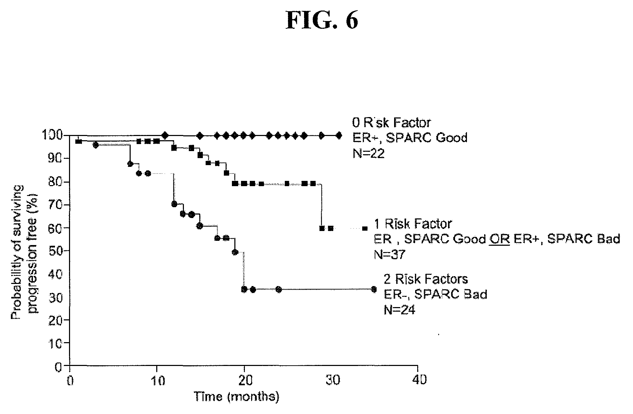

The addition of SPARC SMS clusters to ER further discriminated patients with low risk (0 risk factors) and high risk (2 risk factors) (log rank p values for different number of risk factors: 0 factors vs 2 factors, p = 0.0001; 1 factor vs 2 factors, p = 0.026.) (FIG. 6).

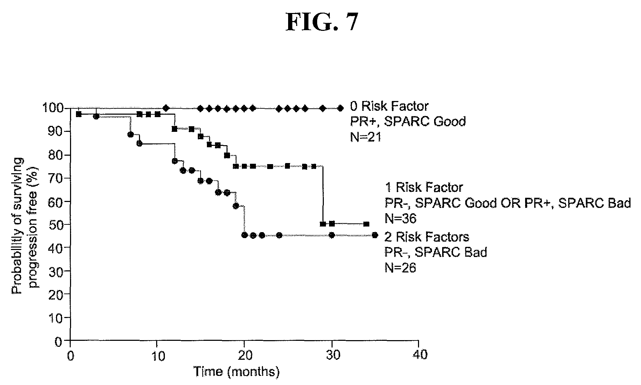

The addition of SPARC SMS clusters to ER further discriminated patients with low risk (0 risk factors) and high risk (2 risk factors) (log rank p values for different number of risk factors: 0 factors versus 2 factors, p = 0.0004; 1 factor versus 2 factors, p = ns.) (FIG. 7). These results demonstrate the combination of the SMS with prior art markers of predictive of response to therapy, progression or death can improve the prognostic accuracy of such markers.

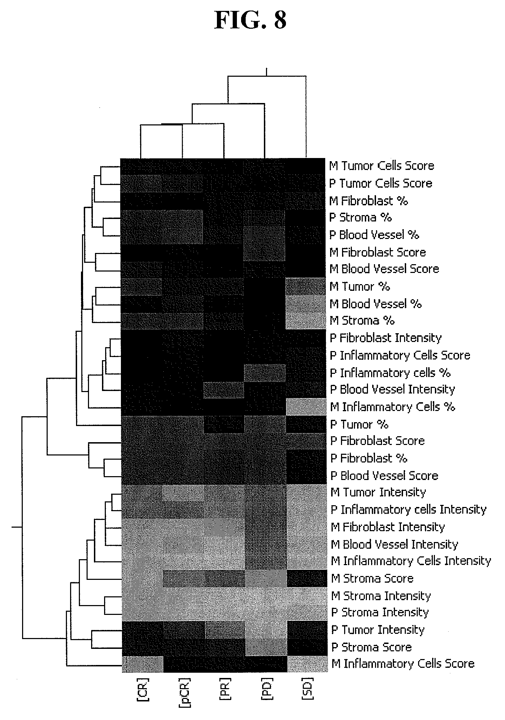

Response was classified as partial complete (pCR), complete response (CR), partial response (PR), (SD), (PD), not available (N/A) (Table 7). The SMS for Response was also identified by cluster analysis (FIG. 8).

**Table 6. Response Groups**

| **Response** | **N** |
|---|---|
| pCR | 9 |
| CR | 9 |
| PR | 54 |
| SD | 5 |
| PD | 2 |
| N/A | 4 |

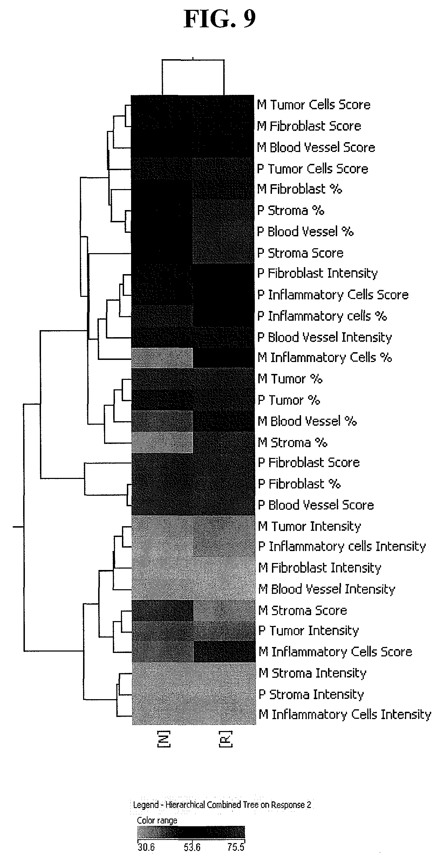

Alternatively, the response outcomes could be shown could be grouped into responders (pCR, CR, PR; n= 72) and nonresponders (SD, PD; n=7). For this binary clasification of Response, the SMS was also identified by cluster analysis (FIG. 9).

The parameters involved in the Response SMS were indentified by cluster analysis (FIG. 9) (Table 7).

**Table 7. Breast Cancer Response SMS Components**

| **SMS Component** | **Cut Off Value** | **p**-**Value** |
|---|---|---|
| M Stroma % | ≥ 60% | 0.002 |
| M Tumor % | ≥ 60% | 0.027 |
| M Blood Vessel % | ≥ 60% | 0.0497 |
| P Tumor % | ≥ 60% | 0.054 |

Data from the CA040 Pancreatic Cancer Trial were also analyzed. Further the analysis could be extended to cytology specimens from the same patients.

Hierarchical clustering was performed on the pancreatic cancer data so that the patients were divided into two groups based on SMS. These groups were analyzed for PFS and OS outcomes. Clustering reveal that SPARC Low Risk components taken together are significantly higher (∼33%) in SPARC (total score 839 vs 629, sum of significant means) than the High Risk components. Individual components across all the compartments examined (Tumor cell, Fibroblast, Inflammatory cells, Blood Vessels, and Acellular stroma) were higher in SPARC for the Low Risk group.

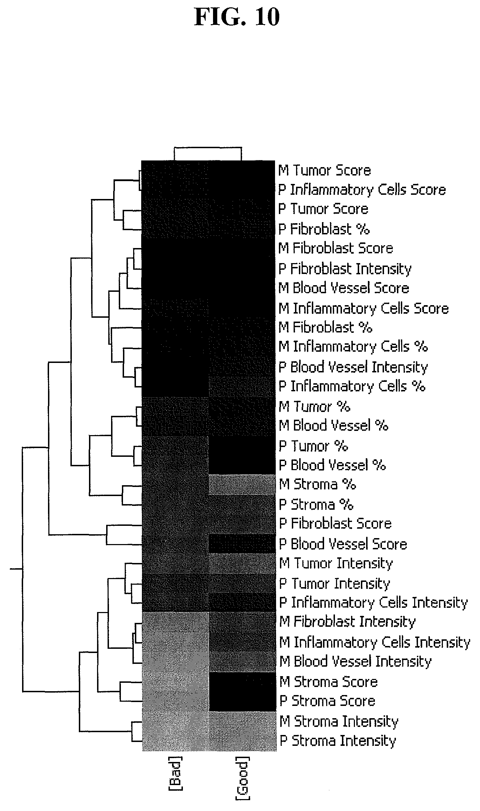

Moreover,using the percent positive cells and quantifying the intensity as 0+=0, 1+=25, 2+=50, 3+=75, 4+=100 and the scores as "negative" = 0, "weakly positive"= 33, "moderately positive"=66 "strongly positive" = 100 gave the following overall results (again, the most important parameters were identified(FIG 10) (Tables 8) and cut off values (Table 9).

**Table 8. Pancreatic Cancer SMS Components**

| **Mean of Variable** | **SPARC High Risk** | **SPARC Low Risk** | **High vs Low Risk cluster p-value** |
|---|---|---|---|
| Poly Fibroblast Score | 65.52 | 86.83 | 1.01E-06 |
| Poly Fibroblast Intensity | 40.63 | 67.81 | 1.57E-04 |
| Poly Tumor Intensity | 25.84 | 48.80 | 2.27E-04 |
| Mab Stroma % | 61.88 | 82.00 | 3.28E-03 |
| Poly Inflammatory Cells Intensity | 25.84 | 42.26 | 4.29E-03 |
| Poly Inflammatory Cells Score | 49.05 | 67.14 | 7.49E-03 |
| Poly Blood Vessel % | 50.94 | 68.00 | 8.09E-03 |
| Poly Tumor Score | 54.72 | 75.55 | 8.10E-03 |
| Poly Blood Vessel Intensity | 32.81 | 45.96 | 9.44E-03 |
| Poly Fibroblast % | 54.06 | 70.63 | 1.37E-02 |
| Poly Blood Vessel Score | 63.52 | 75.03 | 2.02E-02 |
| Poly Inflammatory Cells % | 42.66 | 58.50 | 2.81 E-02 |
| Poly Stroma Score | 61.88 | 50.55 | 5.00E-02 |
| Mab Stroma Intensity | 21.63 | 16.63 | 8.64E-02 |
| Poly Tumor % | 56.56 | 69.75 | 1.06E-01 |
| Mab Fibroblast Intensity | 25.83 | 32.94 | 1.37E-01 |
| Mab Tumor Intensity | 28.16 | 35.73 | 1.63E-01 |
| Mab Stroma Score | 46.56 | 37.91 | 1.99E-01 |
| Poly Stroma % | 69.06 | 78.00 | 2.51E-01 |
| Mab Blood Vessel Intensity | 22.39 | 24.90 | 4.50E-01 |
| Mab Inflammatory Cells Intensity | 24.31 | 27.03 | 4.54E-01 |
| Mab Fibroblast Score | 56.77 | 53.00 | 5.58E-01 |
| Mab Blood Vessel Score | 52.20 | 48.94 | 6.09E-01 |
| Mab Fibroblast % | 55.78 | 52.38 | 6.31E-01 |
| Mab Tumor % | 65.63 | 63.00 | 7.30E-01 |
| Poly Stroma Intensity | 26.56 | 25.85 | 8.60E-01 |
| Mab Tumor Score | 58.88 | 59.99 | 8.74E-01 |
| Mab Inflammatory Cells Score | 47.53 | 46.81 | 9.04E-01 |
| Mab Blood Vessel % | 59.06 | 58.25 | 9.05E-01 |
| Mab Inflammatory Cells % | 46.88 | 47.38 | 9.47E-01 |
| **Sum of all means** | **1393.11** | **1617.51** | |

Thus, the cut off values were

**Table 9. Pancreatic Cut Offs (using components with significant p-values)**

| **Component** | **Cut Off Value High Risk** | **Cut Off Value Low Risk** |
|---|---|---|
| P Fibroblast Score | ≤66 | >87 |
| P Fibroblast Intensity | <41 | >68 |
| P Tumor Intensity | <26 | ≥49 |
| M Stroma% | <49 | ≥82 |
| P Inflammatory Cells Intensity | 51 | 42 |
| P Inflammatory Cells Score | <55 | ≥67 |
| P Blood Vessle % | <33 | ≥68 |
| P Tumor Score | ≤54 | >76 |
| P Blood Vessel Intensity | 64 | 46 |
| P Fibroblast% | ≤54 | >71 |
| P Blood Vessel Intensity | <64 | ≥75 |
| P Inflammatory Cells % | ≤43 | ≥59 |
| P Stroma Score | ≤62 | ≥51 |

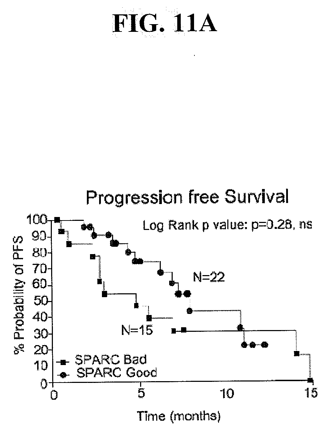

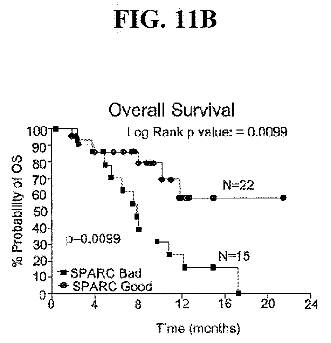

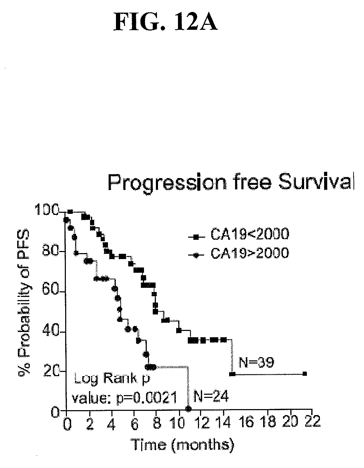

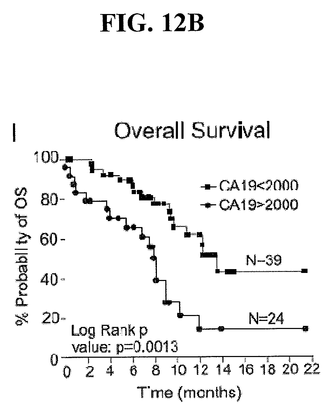

The SMS could distinguish good outcome from bad for OS, but not PFS (FIG. 11 A and B). CA19-9 level is a known risk factor for rapid progression in pancreatic cancer and in the trial CA19-9 level was able to separate PFS and OS groups (FIG. 12). However, there was no correlation between the risk factors SPARC Bad and CA19-9 ≥ 2000 U/ml. Accordingly, SPARC and CA 19-1 were found to be independent prognostic factors for overall survival (Table 10).

**Table 10. SMS for Pancreatic Cancer Is Independent of CA 19-9**

| | CA19-9 < 2000 U/ml | CA19-9 ≥ 2000 U/ml | statistics |
|---|---|---|---|
| Distribution of Pts with SPARC Bad signature in CA19-9 groups | 7/20 (35%) | 6/15 (40%) | *p* = *ns* |

| | SPARC Bad | SPARC Good | statistics |
|---|---|---|---|
| Distribution of Pts with CA19-9 ≥ 2000 U/ml in SPARC clusters | 6/13 (46%) | 9/22 (41%) | *p = ns* |

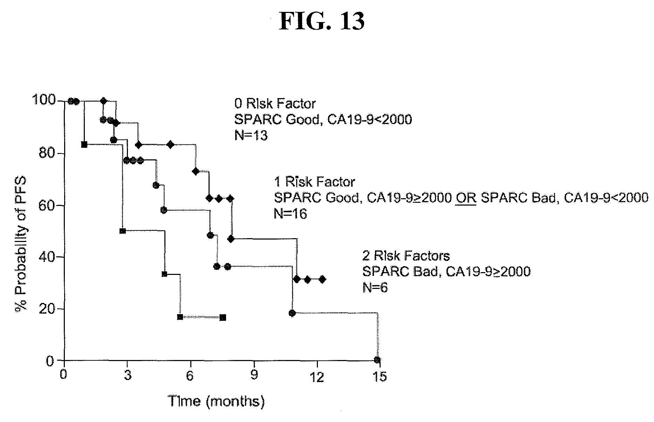

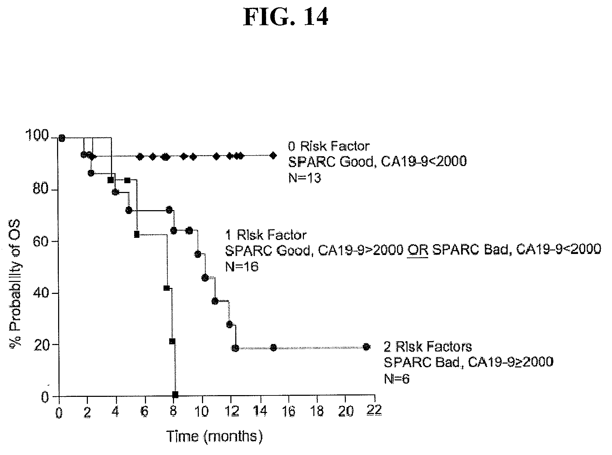

Surprisingly, SMS combined with CA 19-1 level improved stratification PFS and OS (FIGS. 13 and 14).

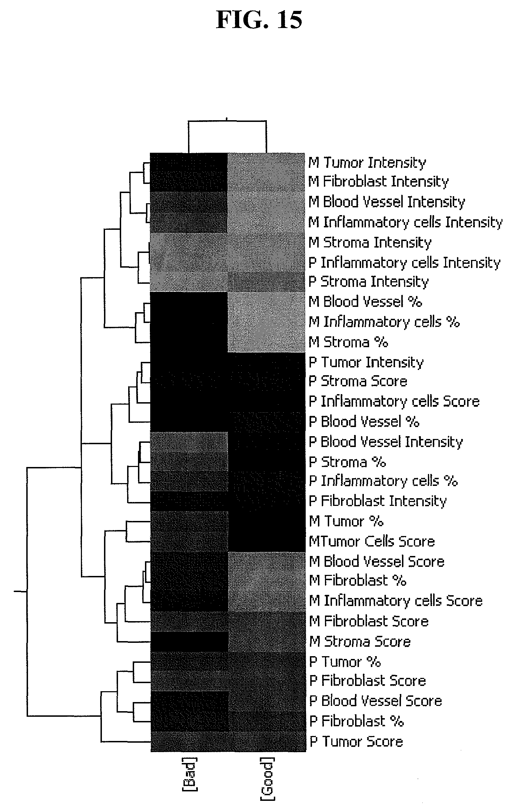

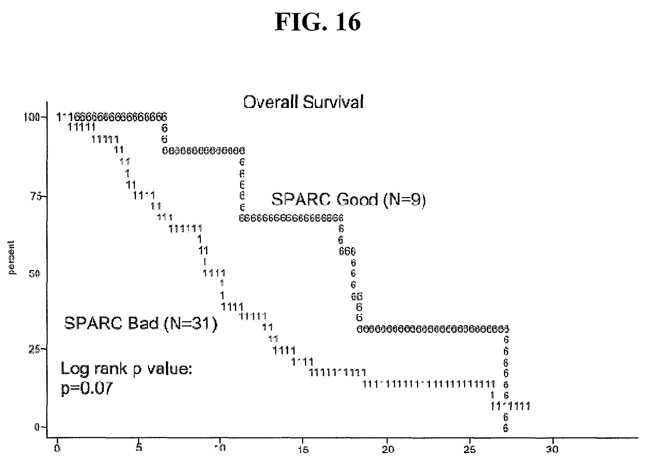

Further analysis of the utility of SMS was undertaken in patients advanced melanoma from the ABX054 Trial. Again, prognostic parameters (Table 11) were identified using hierarchical clustering (FIGs. 15 and 16).

**Table 11. Melanoma PFS Prognostic Parameters**

| **SMS Component** | **Cut Off Value** | **p value** |
|---|---|---|
| M Blood Vessel % | ≤ 50% | <0.0001 |
| M Stroma Score | slightly positive | <0.0001 |
| M Inflammatory cells % | ≤ 50% | <0.0001 |
| M Blood Vessel Score | slightly positive | <0.0001 |
| M Inflammatory cells Score | slightly positive | <0.0001 |
| M Stroma % | ≤ 50% | <0.0001 |
| M Fibroblast Intensity | 1+ to 2+ | <0.0001 |
| M Blood Vessel Intensity | 1+ to 2+ | 0.0006 |
| M Tumor Intensity | 1+ to 2+ | 0.0007 |
| M Tumor Cells Score | slightly positive | 0.0021 |
| M Fibroblast % | ≤ 50% | 0.0022 |
| M Inflammatory cells Intensity | 1+ to 2+ | 0.0029 |
| M Fibroblast Score | slightly positive | 0.0036 |
| M Tumor % | ≤ 50% | 0.0205 |

### EXAMPLE 3

This is an prophetic example of the use of a k-means clustering to generate an SMS and its use to classify individuals into risk groups.

First the centroids for each SMS component must be defined using a training set. Consider a hypothetical data set consisting of the scores of two components of the SMS, e.g., M% tumor and P% Tumor on each of seven individuals:

**Table 12.**

| Subject | M% Tumor | P% Tumor |
|---|---|---|
| **1** | **10** | **10** |
| 2 | 15 | 20 |
| 3 | 30 | 40 |
| **4** | **50** | **70** |
| 5 | 35 | 50 |
| 6 | 45 | 50 |
| 7 | 5 | 45 |

This data set is to be grouped into two clusters, e.g., responder and non-responder. As a first step in finding a sensible initial partition, let the M% tumor and P% Tumor values of the two individuals furthest apart (using the Euclidean distance measure) and with known different responses, define the initial cluster means, giving:

**Table 13.**

| | Individual | Mean Vector (centroid) |
|---|---|---|
| Responder Cluster | 1 | (10, 10) |
| Nonresponder Cluster | 4 | (50, 70) |

The remaining individuals are now examined in sequence and allocated to the cluster to which they are closest, in terms of Euclidean distance to the cluster mean. The mean vector is recalculated each time a new member is added. This leads to the following series of steps:

**Table 14.**

| | Responder Cluster | | Nonresponder Cluster | |
|---|---|---|---|---|
| Step | Individual | Mean Vector (centroid) | Individual | Mean Vector (centroid) |
| 1 | 1 | (10, 10) | 4 | (50, 70) |
| 2 | 1, | (12, 15) | 4 | (50, 70) |
| 3 | 1, 2, 3 | (18, 23) | 4 | (50, 70) |
| 4 | 1, 2, 3 | (18, 23) | 4, 5 | (42, 60) |
| 5 | 1, 2, 3 | (18, 23) | 4, 5, 6 | (43, 57) |
| 6 | 1, 2, 3 | (18, 23) | 4, 5, 6, 7 | (41, 54) |

Now the initial partition has changed, and the two clusters at this stage have the following characteristics:

**Table 15.**

| | Individual | Mean Vector (centroid) |
|---|---|---|
| Responder | 1, 2, 3 | (18, 23) |
| Nonresponder | 4, 5, 6, 7 | (41, 54) |

One cannot yet be sure that each individual has been assigned to the right cluster either mathematically or in the real world. The next step looks at the mathematical quality of the clusters by comparing each individual's distance to its own cluster mean and to that of the opposite cluster, resulting in:

**Table 16.**

| Individual | Distance to mean (centroid) of Responder Cluster | Distance to mean (centroid) of Nonrespond Cluster |
|---|---|---|
| 1 | 15 | 54 |
| 2 | 04 | 43 |
| 3 | 21 | 18 |
| 4 | 57 | 18 |
| 5 | 32 | 07 |
| 6 | 38 | 06 |
| 7 | 28 | 11 |

Only individual 3 is nearer to the mean of the opposite cluster than its own. In other words, each individual's distance to its own cluster mean should be smaller that the distance to the other cluster's mean (which is not the case with individual 3). Thus, individual 3 is relocated to the other cluster resulting in the new partition:

**Table 17.**

| | Individual | Mean Vector (centroid) |
|---|---|---|
| Responder | 1, 2 | (13, 15) |
| Nonresponder | 3, 4, 5, 6, 7 | (39, 51) |

This followed by the relocation of individuals based on response, which is again tested mathematically. The iterative relocation would now continue from this new partition until no more relocations occur. However, in this example each individual is now nearer its own cluster mean than that of the other cluster and the iteration stops, choosing the latest partitioning as the final cluster solution.

Any new individuals could then be classified as a responder or a nonresponder based on which centroid they are closer to.

In addition, although two components were used throughout this example, after the training set has been processed, the components which are most discriminative could be determined by any suitable method and only those components used to define the centroids and classify new individuals.

The use of the terms "a" and "an" and "the" and similar referents in the context of describing the invention (especially in the context of the following claims) are to be construed to cover both the singular and the plural, unless otherwise indicated herein or clearly contradicted by context. The terms "comprising," "having," "including," and "containing" are to be construed as open-ended terms (i.e., meaning "including, but not limited to,") unless otherwise noted. Recitation of ranges of values herein are merely intended to serve as a shorthand method of referring individually to each separate value falling within the range, unless otherwise indicated herein, and each separate value is incorporated into the specification as if it were individually recited herein. All methods described herein can be performed in any suitable order unless otherwise indicated herein or otherwise clearly contradicted by context. The use of any and all examples, or exemplary language (e.g., "such as") provided herein, is intended merely to better illuminate the invention and does not pose a limitation on the scope of the invention unless otherwise claimed. No language in the specification should be construed as indicating any non-claimed element as essential to the practice of the invention.
Preferred embodiments of this invention are described herein, including the best mode known to the inventors for carrying out the invention. Variations of those preferred embodiments may become apparent to those of ordinary skill in the art upon reading the foregoing description. The inventors expect skilled artisans to employ such variations as appropriate, and the inventors intend for the invention to be practiced otherwise than as specifically described herein. Accordingly, this invention includes all modifications and equivalents of the subject matter recited in the claims appended hereto as permitted by applicable law. Moreover, any combination of the above-described elements in all possible variations thereof is encompassed by the invention unless otherwise indicated herein or otherwise clearly contradicted by context

## Claims

1. A chemotherapeutic agent for use in a chemotherapeutic regimen for treating a breast cancer or pancreatic cancer in a mammal, wherein the chemotherapeutic agent is to be administered to the mammal if the cancer SPARC microenvironment signature (SMS) satisfies the criteria of a predefined SMS, wherein the SMS is determined upon
a. preparing a plurality of histologic sections of the cancer for immunohistology;
b. immunostaining one or more of the histologic sections of the cancer with a first anti-SPARC antibody, wherein the first anti-SPARC antibody preferentially stains SPARC in cancer cells;
c. immunostaining one or more of the histologic sections of the cancer with a second anti-SPARC antibody, wherein the second anti-SPARC antibody preferentially stains SPARC in fibroblasts;
d. determining the staining of cancer cells, fibroblasts, inflammatory cells, acellular stroma/matrix, blood vessels, nerve tissue, and normal anatomy within the cancer, or any combinations thereof, with the first anti-SPARC antibody and the staining of cancer cells, fibroblasts, inflammatory cells, acellular stroma/matrix, blood vessels, nerve tissue, and normal anatomy within the cancer, or any combinations thereof, with the second antibody, thereby determining a SPARC microenvironment signature (SMS),
and wherein the chemotherapeutic agent is nab-paclitaxel.

2. An *in vitro* method for predicting the response of a breast cancer or a pancreatic cancer in a mammal to a chemotherapeutic regimen comprising:
a. preparing a plurality of histologic sections of the cancer to obtain a SPARC microenvironment signature (SMS);
b. immunostaining one or more of the histologic sections of the cancer with a first anti-SPARC antibody, wherein the first anti-SPARC antibody preferentially stains SPARC in cancer cells;
c. immunostaining one or more of the histologic sections of the cancer with a second anti-SPARC antibody, wherein the second anti-SPARC antibody preferentially stains SPARC in fibroblasts;
d. determining the staining of cancer cells, fibroblasts, inflammatory cells, acellular stroma/matrix, blood vessels, nerve tissue, and normal anatomy within the cancer, or any combinations thereof, with the first anti-SPARC antibody and the staining of cancer cells, fibroblasts, inflammatory cells, acellular stroma/matrix, blood vessels, nerve tissue, and normal anatomy within the cancer, or any combinations thereof, with the second antibody;
e. predicting a positive response to the chemotherapeutic regimen if a predefined SMS is demonstrated by the immunostaining,
and wherein the chemotherapeutic regimen comprised nab-paclitaxel.

3. An *in vitro* method of predicting if a mammal with a breast cancer has a low risk of the progression of that cancer comprising:
a. preparing a plurality of histologic sections of the cancer to obtain a SPARC microenvironment signature (SMS);
b. immunostaining one or more of the histologic sections of the cancer with a first anti-SPARC antibody, wherein the first anti-SPARC antibody preferentially stains SPARC in cancer cells;
c. immunostaining one or more of the histologic sections of the cancer with a second anti-SPARC antibody, wherein the second anti-SPARC antibody preferentially stains SPARC in fibroblasts;
d. determining the staining of cancer cells, fibroblasts, inflammatory cells, acellular stroma/matrix, blood vessels, nerve tissue, and normal anatomy within the cancer, or any combinations thereof, with the first anti-SPARC antibody and the staining of cancer cells, fibroblasts, inflammatory cells, acellular stroma/matrix, blood vessels, nerve tissue, and normal anatomy within the cancer, or any combinations thereof, with the second antibody; and
e. determining that there is a low risk of progression if the cancer SMS satisfies the criteria of a predefined SMS.

4. The chemotherapeutic agent for use according to claim 1 or the method according to claim 2, wherein the cancer is a pancreatic cancer.

5. The chemotherapeutic agent for use according to claim 1 or the method according to claim 2, wherein the cancer is a breast cancer.

6. The chemotherapeutic agent for use according to claim 1, 4 or 5, or the method according to claim 2, 3, 4 or 5, wherein the mammal is a human.

7. A therapeutic agent for use in a therapy for treating a breast cancer or pancreatic cancer from a first mammal, wherein the therapeutic agent is to be administered to the first mammal if the cancer from the first mammal's SMS maps to an outcome group that responds to the therapy, wherein the cancer from the first mammal is classified as a member of the outcome group upon
a. determining two or more predictive SMS's for the therapy comprising:
i. preparing a plurality of histologic sections of cancer from other mammals with known outcomes for the therapy;
ii. immunostaining one or more of the histologic sections of each of the cancer from the other mammals with known outcomes for the therapy, with a first anti-SPARC antibody, wherein the first anti-SPARC antibody preferentially stains SPARC in cancer cells;
iii. immunostaining one or more of the histologic sections of each of the cancer from the other mammals with known outcomes for the therapy, with a second anti-SPARC antibody, wherein the second anti-SPARC antibody preferentially stains SPARC in fibroblasts;
iv. determining the immunostaining pattern of each of the cancers from other mammals with known outcomes for the therapy, for cancer cells, fibroblasts, inflammatory cells, acellular stroma/matrix, blood vessels, nerve tissue, and normal anatomy within the cancers from other mammals with known outcomes for the therapy, or any combinations thereof, with the first anti-SPARC antibody and the immunostaining of cancer cells, fibroblasts, inflammatory cells, acellular stroma/matrix, blood vessels, nerve tissue, and normal anatomy within the cancer from other mammals with known outcomes for the therapy, or any combinations thereof, with the second antibody, thereby determining the SMS of each cancer from the other mammals with known outcomes for the therapy;
v. clustering the cancer SMS's of each cancer from the other mammals with known outcomes for the therapy into two or more outcome groups, wherein the SMS centroid of each outcome group defines a predictive SMS;
b. determining the SMS of the cancer from the first mammal by a process comprising:
i. preparing a plurality of histologic sections of the cancer from the first mammal;
ii. immunostaining one or more of the histologic sections of the cancer from the first mammal with a first anti-SPARC antibody, wherein the first anti-SPARC antibody preferentially stains SPARC in cancer cells;
iii. immunostaining one or more of the histologic sections of the cancer from the first mammal with a second anti-SPARC antibody, wherein the second anti-SPARC antibody preferentially stains SPARC in fibroblasts,
iv. determining the immunostaining pattern of the cancer in the first mammal for cancer cells, fibroblasts, inflammatory cells, acellular stroma/matrix, blood vessels, nerve tissue, and normal anatomy within the cancer from the first mammal, or any combinations thereof, with the first anti-SPARC antibody and the immunostaining of the cancer from the first mammal of cancer cells, fibroblast, inflammatory cells, acellular stroma/matrix, blood vessels, nerve tissue, and normal anatomy within the cancer from the first mammal, or any combinations thereof, with the second antibody, thereby determining the first mammal's cancer SMS;
c. determining the Euclidian distance of the cancer from the first mammal's SMS to the predictive SMS's determined in (a) and classifying the cancer from the first mammal as a member of the outcome group with the closest predictive SMS,
and wherein the cancer from the first mammal is a breast cancer or a pancreatic cancer and the therapeutic agent is nab-paclitaxel.

8. The therapeutic agent for use according to claim 7, wherein the cancers from the first mammal is of the same type as at the cancers from other mammals with known outcomes for the therapy.

9. The therapeutic agent for use according to claim 7, wherein the cancer from the first mammal is a pancreatic cancer.

10. The therapeutic agent for use according to claim 7, wherein the clustering of the cancer SMS's of each cancer from the other mammals with known outcomes for the therapy is performed by one or more of K-means, Self Organizing Maps and Hierarchical clustering.

11. The therapeutic agent for use according to claim 7, wherein the cancer from the first mammal is a breast cancer.

## Patentansprüche

1. Ein Chemotherapeutikum zur Verwendung bei einem chemotherapeutischen Behandlungsschema zur Behandlung von Brust- oder Pankreaskrebs in einem Säuger, bei dem das Chemotherapeutikum dem Säuger verabreicht werden soll, wenn die Signatur der SPARC-Mikroumgebung (SMS) des Krebses die Kriterien einer vorher definierten SMS erfüllt, wobei die SMS bestimmt wird durch:
a. Herstellen einer Vielzahl von histologischen Schnitten des Krebses für die Immunhistologie;
b. Immunfärbung von einem oder mehreren der histologischen Schnitte des Krebses mit einem ersten anti-SPARC-Antikörper, worin der erste anti-SPARC-Antikörper bevorzugt SPARC in Krebszellen anfärbt;
c. Immunfärbung von einem oder mehreren der histologischen Schnitte des Krebses mit einem zweiten anti-SPARC-Antikörper, worin der zweite anti-SPARC-Antikörper bevorzugt SPARC in Fibroblasten anfärbt;
d. Bestimmen der Färbung der Krebszellen, Fibroblasten, inflammatorischen Zellen, azellulären Stroma/Matrix, Blutgefäße, Nervengewebe und normalen Anatomie innerhalb des Krebses, oder jeder Kombination, davon mit dem ersten anti-SPARC-Antikörper und der Färbung der Krebszellen, Fibroblasten, inflammatorischen Zellen, azellulären Stroma/Matrix, Blutgefäße, Nervengewebe und normalen Anatomie innerhalb des Krebses, oder jeder Kombination davon, mit dem zweiten Antikörper, wodurch die Signatur der SPARC-Mikroumgebung (SMS) bestimmt wird,
und worin das Chemotherapeutikum nab-Paclitaxel ist.

2. *In vitro*-Verfahren zur Vorhersage des Ansprechens eines Brust- oder Pankreaskrebses in einem Säuger auf ein chemotherapeutisches Behandlungsschema, umfassend:
a. Herstellen einer Vielzahl von histologischen Sektionen des Krebses, um eine Signatur der SPARC-Mikroumgebung (SMS) zu erhalten;
b. Immunfärbung von einem oder mehreren der histologischen Schnitte des Krebses mit einem ersten anti-SPARC-Antikörper, worin der erste anti-SPARC-Antikörper bevorzugt SPARC in Krebszellen anfärbt;
c. Immunfärbung von einem oder mehreren der histologischen Schnitte des Krebses mit einem zweiten anti-SPARC-Antikörper, worin der zweite anti-SPARC-Antikörper bevorzugt SPARC in Fibroblasten anfärbt;
d. Bestimmen der Färbung der Krebszellen, Fibroblasten, inflammatorischen Zellen, azellulären Stroma/Matrix, Blutgefäße, Nervengewebe und normalen Anatomie innerhalb des Krebses, oder jeder Kombination davon, mit dem ersten anti-SPARC-Antikörper und der Färbung der Krebszellen, Fibroblasten, inflammatorischen Zellen, azellulären Stroma/Matrix, Blutgefäße, Nervengewebe und normalen Anatomie innerhalb des Krebses, oder jeder Kombination davon, mit dem zweiten Antikörper;
e. Vorhersage des positiven Ansprechens auf ein chemotherapeutisches Behandlungsschema, wenn eine vorher bestimmte SMS über die Immunfärbung gezeigt wurde,
und wobei das Chemotherapeutikum nab-Paclitaxel ist.

3. *In vitro*-Verfahren zur Vorhersage, ob ein Säuger mit einem Brustkrebs ein niedriges Risiko hat, dass der Krebs voranschreitet, umfassend:
a. Herstellen einer Vielzahl von histologischen Schnitten des Krebses, um eine Signatur der SPARC-Mikroumgebung (SMS) zu erhalten;
b. Immunfärbung von einem oder mehreren der histologischen Schnitte des Krebses mit einem ersten anti-SPARC-Antikörper, worin der erste anti-SPARC-Antikörper bevorzugt SPARC in Krebszellen anfärbt;
c. Immunfärbung von einem oder mehreren der histologischen Schnitte des Krebses mit einem zweiten anti-SPARC-Antikörper, worin der zweite anti-SPARC-Antikörper bevorzugt SPARC in Fibroblasten anfärbt;
d. Bestimmen der Färbung der Krebszellen, Fibroblasten, inflammatorischen Zellen, azellulären Stroma/Matrix, Blutgefäße, Nervengewebe und normalen Anatomie innerhalb des Krebses, oder jeder Kombination davon, mit dem ersten anti-SPARC-Antikörper und der Färbung der Krebszellen, Fibroblasten, inflammatorischen Zellen, azellulären Stroma/Matrix, Blutgefäße, Nervengewebe und normalen Anatomie innerhalb des Krebses, oder jeder Kombination davon, mit dem zweiten Antikörper; und
e. Feststellen, dass ein niedriges Risiko des Fortschreitens besteht, wenn die SMS des Krebses die Kriterien einer vorher definierten SMS erfüllt.

4. Das Chemotherapeutikum zur Verwendung nach Anspruch 1 oder 2, wobei der Krebs Pankreaskrebs ist.

5. Das Chemotherapeutikum zur Verwendung nach Anspruch 1 oder 2, wobei der Krebs Brustkrebs ist.

6. Das Chemotherapeutikum zur Verwendung nach einem der Ansprüche 1-5, wobei der Säuger ein Mensch ist.

7. Ein therapeutisches Mittel zur Verwendung bei einer Therapie zur Behandlung von Brust- oder Pankreaskrebs eines ersten Säugers, wobei das therapeutische Mittel dem ersten Säuger verabreicht werden soll, wenn die SMS des Krebses des ersten Säugers mit der Gruppe mit dem Resultat, dass sie auf die Therapie anspricht, übereinstimmt, wobei der Krebs des ersten Säugers als Mitglied der Gruppe mit diesem Resultat klassifiziert wird durch:
a. Bestimmen von zwei oder mehr vorhersagenden SMS für die Therapie umfassend:
i. Herstellen einer Vielzahl von histologischen Schnitten des Krebses eines Säugers mit bekanntem Resultat für die Therapie;
ii. Immunfärbung von einem oder mehreren der histologischen Schnitte jedes Krebses der anderen Säuger mit bekanntem Resultat für die Therapie mit einem ersten anti-SPARC-Antikörper, worin der erste anti-SPARC-Antikörper bevorzugt SPARC in Krebszellen anfärbt;
iii. Immunfärbung von einem oder mehreren der histologischen Schnitte jedes Krebses der anderen Säuger mit bekanntem Resultat für die Therapie mit einem zweiten anti-SPARC-Antikörper, worin der zweite anti-SPARC-Antikörper bevorzugt SPARC in Fibroblasten anfärbt;
iv. Bestimmung des Musters der Immunfärbung für jeden der Krebse der anderen Säuger mit bekannten Resultaten für die Therapie für Krebszellen, Fibroblasten, inflammatorischen Zellen, azellulären Stroma/Matrix, Blutgefäße, Nervengewebe und normalen Anatomie innerhalb des Krebses der anderen Säuger mit bekannten Resultaten für die Therapie, oder einer Kombination davon, mit dem ersten anti-SPARC-und der Färbung der Krebszellen, Fibroblasten, inflammatorischen Zellen, azellulären Stroma/Matrix, Blutgefäße, Nervengewebe und normalen Anatomie innerhalb des Krebses der anderen Säuger mit bekanntem Resultat für die Therapie mit dem zweiten Antikörper, wodurch die SMS für jeden Krebs der anderen Säuger mit bekanntem Resultat für die Therapie bestimmt wird;
v. Gruppierung der Krebs-SMS in zwei oder mehr Ergebnisgruppen für jeden Krebs der anderen Säuger mit bekanntem Resultat für die Therapie, wobei der Schwerpunkt der SMS für jede Ergebnisgruppe eine vorhersagbare SMS definiert;
b. Bestimmung der SMS des Krebses eines ersten Säugers durch ein Verfahren umfassend:
i. Herstellen einer Vielzahl von histologischen Schnitten des Krebses eines ersten Säugers;
ii. Immunfärbung von einem oder mehreren der histologischen Schnitte des Krebses eines ersten Säugers mit einem ersten anti-SPARC-Antikörper, worin der erste anti-SPARC-Antikörper bevorzugt SPARC in Krebszellen anfärbt;
iii. Immunfärbung von einem oder mehreren der histologischen Schnitte des Krebses eines ersten Säugers mit einem zweiten anti-SPARC-Antikörper, worin der zweite anti-SPARC-Antikörper bevorzugt SPARC in Fibroblasten anfärbt,
iv. Bestimmung des Musters der Immunfärbung des Krebses eines ersten Säugers von Krebszellen, Fibroblasten, inflammatorischen Zellen, azellulären Stroma/Matrix, Blutgefäße, Nervengewebe und normalen Anatomie innerhalb des Krebses des ersten Säugers, oder einer Kombination davon, mit dem ersten anti-SPARC-Antikörper und der Färbung der Krebszellen, Fibroblasten, inflammatorischen Zellen, azellulären Stroma/Matrix, Blutgefäße, Nervengewebe und normalen Anatomie innerhalb des Krebses des ersten Säugers mit dem zweiten Antikörper, wodurch die SMS des ersten Säugers bestimmt wird;
c. Bestimmung des euklidischen Abstands der SMS des Krebses des ersten Säugers in (a) und Klassifizieren des Krebses des ersten Säugers als Mitglied der Ergebnisgruppe mit der am nächsten vorhersagbaren SMS,
und wobei der Krebs des ersten Säugers ein Brust- oder Pankreaskrebs ist und das therapeutische Mittel nab-Paclitaxel ist.

8. Das Chemotherapeutikum zur Verwendung nach Anspruch 7, wobei der Krebs des ersten Säugers vom gleichen Typ ist wie der Krebs von anderen Säugern mit bekanntem Resultat für die Therapie.

9. Das Chemotherapeutikum zur Verwendung nach Anspruch 7, wobei der Krebs des ersten Säugers Pankreaskrebs ist.

10. Das Chemotherapeutikum zur Verwendung nach Anspruch 7, wobei das Gruppieren der SMS des Krebses für jeden Krebs der anderen Säuger mit bekanntem Resultat für die Therapie durch eines oder mehrere aus K-Maßnahmen, selbst-organisierenden Karten und hierarchischen Gruppierungen durchgeführt wird.

11. Das Chemotherapeutikum zur Verwendung nach Anspruch 7, wobei der Krebs des ersten Säugers Brustkrebs ist.

## Revendications

1. Agent chimiothérapeutique à utiliser dans un régime chimiothérapeutique de traitement d'un cancer du sein ou d'un cancer du pancréas chez un mammifère, l'agent chimiothérapeutique devant être administré au mammifère si la signature du microenvironnement de la SPARC (SMS) satisfait le critère d'une SMS prédéfinie, la SMS étant déterminée à l'issue de
a. la préparation d'une pluralité de coupes histologiques du cancer pour immunohistologie ;
b. l'immunocoloration d'une ou plusieurs coupes histologiques du cancer avec un premier anticorps anti-SPARC, le premier anticorps anti-SPARC colorant de manière préférentielle la SPARC dans les cellules cancéreuses ;
c. l'immunocoloration d'une ou plusieurs coupes histologiques du cancer avec un second anticorps anti-SPARC, le second anticorps anti-SPARC colorant de manière préférentielle la SPARC dans les fibroblastes ;
d. la détermination de la coloration des cellules cancéreuses, des fibroblastes, des cellules inflammatoires, du stroma/de la matrice acellulaire, des vaisseaux sanguins, du tissu nerveux, et de l'anatomie normale à l'intérieur du cancer, ou de n'importe quelles combinaisons de ceux-ci, avec le premier anticorps anti-SPARC et la coloration des cellules cancéreuses, des fibroblastes, des cellules inflammatoires, du stroma/de la matrice acellulaire, des vaisseaux sanguins, du tissu nerveux, et de l'anatomie normale à l'intérieur du cancer, ou de n'importe quelles combinaisons de ceux-ci, au second anticorps, déterminant de là une signature du microenvironnement de la SPARC (SMS),
et l'agent chimiothérapeutique étant le nab-paclitaxel.

2. Procédé *in vitro* de prédiction de la réponse d'un cancer du sein ou d'un cancer du pancréas chez un mammifère à un régime chimiothérapeutique comprenant :
a. la préparation d'une pluralité de coupes histologiques du cancer pour obtenir une signature de microenvironnement de la SPARC (SMS) ;
b. l'immunocoloration d'une ou plusieurs coupes histologiques du cancer avec un premier anticorps anti-SPARC, le premier anticorps anti-SPARC colorant de manière préférentielle la SPARC dans les cellules cancéreuses ;
c. l'immunocoloration d'une ou plusieurs coupes histologiques du cancer avec un second anticorps anti-SPARC, le second anticorps anti-SPARC colorant de manière préférentielle la SPARC dans les fibroblastes ;
d. la détermination de la coloration des cellules cancéreuses, des fibroblastes, des cellules inflammatoires, du stroma/de la matrice acellulaire, des vaisseaux sanguins, du tissu nerveux, et de l'anatomie normale à l'intérieur du cancer, ou de n'importe quelles combinaisons de ceux-ci, avec le premier anticorps anti-SPARC et la coloration des cellules cancéreuses, des fibroblastes, des cellules inflammatoires, du stroma/de la matrice acellulaire, des vaisseaux sanguins, du tissu nerveux, et de l'anatomie normale à l'intérieur du cancer, ou de n'importe quelles combinaisons de ceux-ci, grâce au second anticorps ;
e. la prédiction d'une réponse positive au régime chimiothérapeutique si une SMS prédéfinie est démontrée par l'immunocoloration,
et le régime chimiothérapeutique comprenant du nab-paclitaxel.

3. Procédé *in vitro* de prédiction du fait qu'un mammifère ayant un cancer du sein présente un risque faible de progression de ce cancer comprenant :
a. la préparation d'une pluralité de coupes histologiques du cancer pour obtenir une signature de microenvironnement de la SPARC (SMS) ;
b. l'immunocoloration d'une ou plusieurs coupes histologiques du cancer avec un premier anticorps anti-SPARC, le premier anticorps anti-SPARC colorant de manière préférentielle la SPARC dans les cellules cancéreuses ;
c. l'immunocoloration d'une ou plusieurs coupes histologiques du cancer avec un second anticorps anti-SPARC, le second anticorps anti-SPARC colorant de manière préférentielle la SPARC dans les fibroblastes ;
d. la détermination de la coloration des cellules cancéreuses, des fibroblastes, des cellules inflammatoires, du stroma/de la matrice acellulaire, des vaisseaux sanguins, du tissu nerveux, et de l'anatomie normale à l'intérieur du cancer, ou de n'importe quelles combinaisons de ceux-ci, avec le premier anticorps anti-SPARC et la coloration des cellules cancéreuses, des fibroblastes, des cellules inflammatoires, du stroma/de la matrice acellulaire, des vaisseaux sanguins, du tissu nerveux, et de l'anatomie normale à l'intérieur du cancer, ou de n'importe quelles combinaisons de ceux-ci, grâce au second anticorps ; et
e. la détermination du fait qu'il existe un faible risque de progression si la SMS du cancer satisfait le critère d'une SMS prédéfinie.

4. Agent chimiothérapeutique à utiliser selon la revendication 1 ou procédé selon la revendication 2, le cancer étant un cancer du pancréas.

5. Agent chimiothérapeutique à utiliser selon la revendication 1 ou procédé selon la revendication 2, le cancer étant un cancer du sein.

6. Agent chimiothérapeutique à utiliser selon la revendication 1, 4 ou 5, ou procédé selon la revendication 2, 3, 4 ou 5, le mammifère étant un être humain.

7. Agent thérapeutique à utiliser dans une thérapie de traitement d'un cancer du sein ou d'un cancer du pancréas d'un premier mammifère, l'agent thérapeutique devant être administré au premier mammifère si le cancer de la SMS du premier mammifère s'applique à un groupe de type d'issues du cancer qui répond à la thérapie, le cancer du premier mammifère étant classé comme membre du groupe de type d'issues du cancer à l'issue de
a. la détermination de deux ou plusieurs SMS prédictives pour la thérapie comprenant :
i. la préparation d'une pluralité de coupes histologiques du cancer provenant d'autres mammifères présentant des résultats connus pour la thérapie ;
ii. l'immunocoloration d'une ou plusieurs coupes histologiques de chaque cancer provenant des autres mammifères présentant des résultats connus pour la thérapie, avec un premier anticorps anti-SPARC, le premier anticorps anti-SPARC colorant de manière préférentielle la SPARC dans les cellules cancéreuses ;
iii. l'immunocoloration d'une ou plusieurs coupes histologiques de chaque cancer provenant des autres mammifères présentant des résultats connus pour la thérapie, avec un second anticorps anti-SPARC, le second anticorps anti-SPARC colorant de manière préférentielle la SPARC dans les fibroblastes ;
iv. la détermination du profil d'immunocoloration de chacun des cancers provenant d'autres mammifères présentant des résultats connus pour la thérapie, pour les cellules cancéreuses, les fibroblastes, les cellules inflammatoires, le stroma/la matrice acellulaire, les vaisseaux sanguins, le tissu nerveux, et l'anatomie normale à l'intérieur des cancers provenant d'autres mammifères présentant des résultats connus pour la thérapie, ou de n'importe quelles combinaisons de ceux-ci, au premier anticorps anti-SPARC et l'immunocoloration des cellules cancéreuses, des fibroblastes, des cellules inflammatoires, du stroma/de la matrice acellulaire, des vaisseaux sanguins, du tissu nerveux, et de l'anatomie normale à l'intérieur du cancer provenant d'autres mammifères présentant des résultats connus pour la thérapie, ou de n'importe quelles combinaisons de ceux-ci, avec le second anticorps, déterminant de là la SMS de chaque cancer des autres mammifères présentant des résultats connus pour la thérapie ;
v. le regroupement des SMS de cancer de chaque cancer provenant des autres mammifères présentant des résultats connus pour la thérapie en deux ou plusieurs groupes de type d'issues du cancer, le centroïde des SMS de chaque groupe de type d'issues du cancer définissant une SMS prédictive ;
b. la détermination de la SMS du cancer provenant du premier mammifère par un procédé comprenant :
i. la préparation d'une pluralité de coupes histologiques du cancer provenant du premier mammifère ;
ii. l'immunocoloration d'une ou plusieurs coupes histologiques du cancer provenant du premier mammifère avec un premier anticorps anti-SPARC, le premier anticorps anti-SPARC colorant de manière préférentielle la SPARC dans les cellules cancéreuses ;
iii. l'immunocoloration d'une ou plusieurs coupes histologiques du cancer provenant du premier mammifère avec un second anticorps anti-SPARC, le second anticorps anti-SPARC colorant de manière préférentielle la SPARC dans les fibroblastes,
iv. la détermination du profil d'immunocoloration du cancer chez le premier mammifère pour les cellules cancéreuses, les fibroblastes, les cellules inflammatoires, le stroma/la matrice acellulaire, les vaisseaux sanguins, le tissu nerveux, et l'anatomie normale à l'intérieur du cancer provenant du premier mammifère, ou de n'importe quelles combinaisons de ceux-ci, avec le premier anticorps anti-SPARC et l'immunocoloration du cancer provenant du premier mammifère de cellules cancéreuses, de fibroblaste, de cellules inflammatoires, du stroma/de la matrice acellulaire, de vaisseaux sanguins, du tissu nerveux, et de l'anatomie normale à l'intérieur du cancer provenant du premier mammifère, ou de n'importe quelles combinaisons de ceux-ci, avec le second anticorps, déterminant de là la SMS de cancer du premier mammifère ;
c. la détermination de la distance euclidienne du cancer à partir de la SMS du premier mammifère jusqu'à la SMS prédictive déterminée dans (a) et la classification du cancer provenant du premier mammifère comme membre du groupe de type d'issues du cancer présentant la SMS prédictive la plus proche, et le cancer provenant du premier mammifère étant un cancer du sein ou un cancer du pancréas et l'agent thérapeutique étant le nab-paclitaxel.

8. Agent thérapeutique à utiliser selon la revendication 7, les cancers provenant du premier mammifère étant du même type que les cancers des autres mammifères ayant des résultats connus pour la thérapie.

9. Agent thérapeutique à utiliser selon la revendication 7, le cancer provenant du premier mammifère étant un cancer du pancréas.

10. Agent thérapeutique à utiliser selon la revendication 7, le regroupement des SMS de cancer de chaque cancer provenant des autres mammifères présentant des résultats connus pour la thérapie étant exécuté par un ou plusieurs parmi les moyens K, les cartes d'auto-organisation et le regroupement hiérarchique.

11. Agent thérapeutique à utiliser selon la revendication 7, le cancer provenant du premier mammifère étant un cancer du sein.
